# EUROPEAN PATENT APPLICATION

(11) **EP 1 650 562 A1**
(43) Date of publication of application: **26.04.2006**
(21) Application number: 04771311.0
(22) Date of filing: 30.07.2004
(51) Int. Cl.: G01N 33/50, G01N 33/15, C07K 14/00, C12N 15/09

(54) **METHOD OF SCREENING USEFUL PROTEIN**

(30) Priority: 31.07.2003 JP 2003205139; 15.12.2003 JP 2003416228
(71) Applicant: Genefield, Inc., Tokyo 1530041 (JP)
(72) Inventor: Nemoto, Naoto;, 4-16, Minami-cho, Toda-shi, Saitama 335- (JP); Yamaguchi, Jun-ichi;, Chiba 277-0861 (JP)
(74) Representative: Dehmel, Albrecht
(86) International application number: PCT/JP2004/011308
(87) International publication number: WO 2005/012902

(57) **Abstract**

Objectives of the present invention are achieved through, for example, methods for identifying useful proteins by synthesizing a protein that randomly comprises a disulfide bond/disulfide bonds, via random introduction of cysteine residues into the amino acid sequence, and analyzing the function of the protein, wherein the method comprises the steps of:
(a) preparing one or more mRNAs encoding a protein that comprise at least two cysteine residues, and linking each of the prepared mRNAs with puromycin or a puromycin-like compound to obtain a mRNA-puromycin conjugate(s);
(b) contacting a translation system with the mRNA-puromycin conjugate(s) obtained in step (a) to synthesize the protein(s), and preparing a mRNA-puromycin-protein conjugate(s); and
(c) contacting one or more target substances with the mRNA-puromycin-protein conjugate(s) prepared in step (b), and determining whether the target substance(s) interacts/interact with any one of the proteins of the mRNA-puromycin-protein conjugate(s).

## Description

### Technical Field

The present invention relates to methods of screening for useful proteins using random cross-linking, proteins obtainable by the screening methods, and such.

### Background Art

Despite efforts for many years, it has proven to be difficult to achieve outstanding results using protein engineering techniques to design proteins with useful functions through structural analysis of proteins. Under the circumstance described above, the concept of "evolutionary molecular engineering" (see Eigen, E. & Gardiner, W., Pure & Appl. Chem., 56, 967-978 (1984)), a molecular designing method based on the principle of evolution, was proposed by *Eigen et al.* in 1984. This led to the development of technologies for selecting molecules with objective functions from a population (library) of various mutants, namely a population of molecules with various structures. In the 1990s, tangible results were obtained by a series of studies called the *"In vitro* evolution" method and the obtained functional molecules vary widely from RNAs, DNAs, proteins, to peptides.

Known methods of screening for nucleic acids (RNAs and DNAs) include: the *in vitro* selection method (see Ellington, A. D. & Szostak, J. W., Nature, 346, 818-822 (1990)); and the systematic evolution of ligands by exponential enrichment (SELEX) method (see Tuerk, C. & Gold, L., Science, 249, 505-510 (1990)). Known methods of screening for peptides and proteins include: the phage display method (see Scott, J. K. & Smith, G. D., Science, 249, 386-390 (1990)); the photolithography (see Fodor, S. P. *et al.,* Science, 251, 767-773 (1991)) in which peptides of various sequences are solid-phase synthesized on a base through photomasking and photoreaction; and the ribosome display method (see Mattheakis, L. C*. et al.*, Proc. Natl. Acad. Sci. USA, 91, 9022-9026) that uses a cell-free translation system.

Furthermore, methods of screening for peptides and proteins that use the *in vitro* virus method (see, for example, Nemoto *et al.,* FEBS Lett. 414, 405 (1997); Tabuchi *et al.,* FEBS Lett. 508, 309 (2001)), more particularly the specific feature of puromycin (see WO 01/016600), have also been developed.

Among methods of screening for peptides and proteins, the ribosome display method and the *in vitro* virus method, which use a cell-free translation system, are superior for efficient large-scale screening and for enabling the synthesis of various proteins. However, following these methods, it can prove difficult to achieve the folding that is essential for the expression of protein function, due to the protein synthesis in so-called liquid phase. For example, peptides or proteins comprising many cysteines tend to form intermolecular S-S bridges which inhibit the folding of each of the proteins.

Meanwhile, the function of a protein mainly depends on its tertiary structure. Specifically, a protein exhibits some kind of physiological function through the forming of a particular structure by a certain portion of the protein due to its folding; and the binding of the portion with a receptor, enzyme, or such to activate or inactivate the receptor, enzyme, or such. Tertiary structure analyses of proteins are being actively performed today. However, such analyses are not easy. For example, it is difficult to prepare proteins at large scale to allow analysis by NMR or X-ray diffraction. In addition, X-ray diffraction requires crystallization of proteins, which also is not easy.

### Disclosure of the Invention

Currently, many efforts are concentrated on tertiary structure and functional analyses of *in vivo* expressed proteins. However, as described above, the tertiary structure analysis can be difficult. It is thus desired to establish a technology that allows simple and efficient screening for proteins with particular functions using proteins with various tertiary structures obtainable by artificial synthesis.

The folding of proteins is extremely important in the screening or functional analysis of proteins. However, correct folding is difficult to achieve, due to the occurrence of the above-described intermolecular interactions during the synthesis. It is preferred to establish techniques in which screening or functional analysis is carried out after the protein folding process is complete. It is also preferred to establish methods for designing proteins with higher activity by altering the structures of proteins obtained by such techniques.

Under the above-described circumstances, an objective of the present inventors is to provide a method that enables the simple and efficient screening for proteins having particular functions using proteins with various tertiary structures that are obtainable by artificial synthesis.

The present inventors developed methods in which useful proteins can be screened by: randomly introducing cysteine residues into amino acid sequences; synthesizing a group (random library) of proteins randomly comprising disulfide bonds (S-S bonds); and analyzing their functions.

The present inventors first prepared "immobilized mRNA-puromycin conjugates" that have the structure in which conjugates of (1) nucleic acid molecules (mRNAs) encoding proteins comprising a cysteine residue/cysteine residues and (2) puromycin that serves as a linker of the mRNAs and its translation products (the proteins) were immobilized onto a solid phase. Then, a random library was prepared, wherein the proteins as the translation products of the mRNAs were also immobilized by presenting the conjugates to a translation system. According to the method described above, proteins can be efficiently synthesized on the solid phase through the contact of the immobilized mRNA-puromycin conjugates with the translation system. The synthesized proteins are immobilized onto the solid phase via puromycin.

Furthermore, the present inventors presented the above-described immobilized random library to a reverse transcription system and thereby constructed a random library in which complementary DNAs (the reverse transcription products of mRNAs in the library) had been conjugated. Generally, RNAs are more stable than DNAs. Hence, the above-described library is very useful for contact with test molecules for functional analyses, including protein interaction analysis and such.

Then, the present inventors successfully folded the proteins in the above-described library by subjecting the library to peptide oxidation and forming one or more S-S bonds. Thus, efficient screening of proteins that form tertiary structures, and that actually exhibit functions, was realized.

Screening for useful proteins can be achieved by continuing the procedures as follows: contacting the random library after folding with target molecules; analyzing the function (for example, binding activity to the target molecule) of each of the proteins in the library; and selecting the proteins with preferred functions.

According to the present invention, when a protein with even the slightest binding activity to a proper target molecule is obtained, a more active protein can be obtained, for example, through random or systematic modification of the amino acid sequence of the protein. Specifically, the protein with enhanced binding activity to the target molecule can be obtained by preparing the above-described "immobilized mRNA-puromycin conjugates", comprising mRNAs encoding altered proteins, and appropriately repeating the above-described procedure several times.

The present inventors selected Cy3 as a target molecule and carried out the screening for functional proteins having binding activity to Cy3 to demonstrate how the above-described methods of the present invention can be actually carried out.

As a result, the present inventors succeeded indeed in obtaining several proteins having binding activity to Cy3. Thus, the methods of the present invention were confirmed to be both useful and actually practicable.

The present invention relates to methods of efficiently and readily screening for proteins with particular functions from proteins with various tertiary structures that are obtainable by artificial synthesis. More specifically, the present invention provides methods of screening for useful proteins, proteins obtained by the screening methods, and the like, as described below.
(1) A method of screening for useful proteins by synthesizing a protein randomly comprising a disulfide bond via the random introduction of cysteine residues into the amino acid sequence of the protein, and analyzing the function of the protein, wherein the method comprises the steps of:
   (a) preparing one or more mRNAs encoding a protein/proteins that comprise at least two cysteine residues, and linking each of the prepared mRNAs with puromycin or a puromycin-like compound to obtain mRNA-puromycin conjugate(s);
   (b) contacting a translation system with the mRNA-puromycin conjugate(s) obtained in step (a) to synthesize the protein/proteins, and preparing mRNA-puromycin-protein conjugate(s); and
   (c) contacting one or more target substances with the mRNA-puromycin-protein conjugate(s) prepared in step (b), and determining whether the target substances interact with any one of the proteins within the mRNA-puromycin-protein conjugate(s).
(2) The method of (1), wherein the mRNA-puromycin conjugate(s) is/are obtained by linking the 3'-terminus of the mRNA(s) to puromycin or the puromycin-like compound via a spacer.
(3) The method of (1) or (2), wherein, in step (a), the mRNA(s) is/are prepared by transcription of DNA(s) encoding the protein(s) comprising two or more cysteine residues.
(4) The method of any one of (1) to (3), wherein the translation system used in step (b) is a cell-free translation system.
(5) The method of any one of (1) to (4), wherein, in step (c), the determination whether the target substance(s) interact with the protein(s) is carried out by evaluating whether the two bind to each other through the affinity column chromatography method or the affinity bead method.
(6) The method of any one of (1) to (5), which further comprises the step of:
   identifying the protein(s) and/or the target substance(s) that have been determined in step (c) to interact to each other.
(7) The method of any one of (1) to (6), which further comprises, after step (c), the step of:
   (d) preparing DNA(s) via reverse transcription of the mRNA(s) of the mRNA-puromycin-protein conjugate(s) comprising the protein(s) that interact with the target substance(s), preparing mRNA(s) having a sequence that is altered by the mutagenesis of the DNA(s), and subjecting the mRNA(s) to step (a) to obtain a protein with enhanced interactive force to the target substance(s).
(8) The method of any one of (1) to (7), wherein the mutagenesis of the DNA is achieved by the error-prone PCR.
(9) The method of any one of (1) to (8), wherein a protein with enhanced interactive force to the target substance(s) is obtained by repeating the steps (a) to (d) several times.
(10) The method of any one of (1) to (9), wherein the mRNA(s) encodes/encode a protein/proteins comprising 8 to 500 amino acid residues.
(11) The method of any one of (1) to (10), wherein the mRNA(s) encodes/encode a protein/proteins comprising 10 to 200 amino acid residues.
(12) The method of any one of (1) to (11), wherein the mRNA(s) encodes/encode a protein/proteins comprising 4 to 10 cysteine residues.
(13) The method of any one of (1) to (12), wherein the mRNA(s) encodes/encode a protein/proteins in which the cysteine residues adjacent to each other are separated in the amino acid sequence with an interval of 2 to 20 residues.
(14) The method of any one of (1) to (13), wherein the mRNA(s) encodes/encode a protein/proteins in which the cysteine residues positioned to the most N-terminal side and the most C-terminal side are separated by 10 to 50 residues.
(15) The method of any one of (1) to (14), wherein the spacer comprises, as the major backbone, polynucleotide, polyethylene, polyethylene glycol, polystyrene, peptide nucleic acid, or a combination thereof.
(16) The method of any one of (1) to (15), wherein the spacer comprises a solid-phase immobilization site and wherein the mRNA-puromycin conjugate(s) is/are linked to a solid phase via the solid-phase immobilization site.
(17) The method of any one of (1) to (16), wherein the solid phase is selected from the group consisting of: styrene bead, glass bead, agarose bead, Sepharose bead, magnetic bead, glass base, silicon base, plastic base, metallic base, glass container, plastic container, and membrane.
(18) The method of (16) or (17), wherein the solid-phase immobilization site in the spacer has a cleavable site, and wherein, in step (b), the method comprises the steps of:
   folding the protein(s) on the solid phase;
   cleaving the cleavable site to cleave the mRNA-puromycin-protein conjugate(s) from the solid phase; and then
   subjecting the cleaved mRNA-puromycin-protein conjugate(s) to step (c).
(19) The method of (18), wherein the spacer is a DNA spacer and wherein the cleavable site is a restriction enzyme recognition site.
(20) A synthetic protein obtainable by the method of any one of (1) to (19), which has 8 to 500 amino acid residues, comprises 2 to 10 cysteine residues for forming disulfide bonds, and has an association constant to the target substance that changes due to oxidation and reduction.
(21) A synthetic protein which has 8 to 500 amino acid residues, comprises 2 to 10 cysteine residues for forming disulfide bonds, and has an association constant to the target substance that changes due to oxidation and reduction.

More preferably, the present invention relates to methods of screening for useful proteins, proteins obtained by the screening methods, and the like, as described below.
[1] A method of screening for useful proteins by synthesizing a protein comprising a disulfide bond via the introduction of cysteine residues into the amino acid sequence of the protein, and analyzing the function of the protein, wherein the method comprises the steps of:
   (a) preparing one or more mRNAs encoding a protein/proteins that comprise at least two cysteine residues, and linking each of the prepared mRNAs with puromycin or a puromycin-like compound to obtain mRNA-puromycin conjugate(s);
   (b) contacting a translation system with the mRNA-puromycin conjugate(s) obtained in step (a) to synthesize the protein/proteins, and preparing mRNA-puromycin-protein conjugate(s); and
   (c) contacting one or more target substances with the mRNA-puromycin-protein conjugate(s) prepared in step (b), and determining whether the target substances interact with any one of the proteins within the mRNA-puromycin-protein conjugate(s).
[2] The method of [1], which further comprises, after step (c), the step of:
   (d) preparing DNA(s) via reverse transcription of the mRNA(s) of the mRNA-puromycin-protein conjugate(s) comprising the protein(s) that interact with the target substance(s), preparing mRNA(s) having a sequence that is altered by the mutagenesis of the DNA(s), and subjecting the mRNA(s) to step (a) to obtain a protein with enhanced interactive force to the target substance(s).
[3] A method of screening for useful proteins by synthesizing a protein comprising a disulfide bond via the introduction of cysteine residues into the amino acid sequence of the protein, and analyzing the function of the protein, wherein the method comprises the steps of:
   (a) preparing one or more mRNAs encoding a protein/proteins that comprise at least two cysteine residues, and linking each of the prepared mRNAs with puromycin or a puromycin-like compound to obtain mRNA-puromycin conjugate(s);
   (b) contacting a translation system with the mRNA-puromycin conjugate(s) obtained in step (a) to synthesize a protein/proteins, and preparing mRNA-puromycin-protein conjugate(s);
   (c) preparing DNA(s) via reverse transcription of the mRNA(s) of the mRNA-puromycin-protein conjugate(s) obtained in step (b), and preparing DNA-puromycin-protein conjugate(s); and
   (d) contacting one or more target substances with the DNA-puromycin-protein conjugate(s) prepared in step (c), and determining whether the target substances interact with any one of the proteins of the DNA-puromycin-protein conjugate(s).
[4] The method of [3], which further comprises, after step (d), the step of:
   (e) preparing mRNA(s) having a sequence that is altered by the mutagenesis of the DNA(s) in the DNA-puromycin-protein conjugate(s) comprising the protein(s) that interacts/interact with the target substance(s), and subjecting the mRNA(s) to step (a) to obtain a protein having enhanced interactive force to the target substance(s).
**[5]** The method of any one of [1] to [4], wherein, in step (a), the mRNA-puromycin conjugate(s) has/have the structure in which the 3'-terminus of the mRNA(s) is linked to puromycin or the puromycin-like compound via a spacer.
[6] The method of any one of [1] to [5], wherein, in step (a), the mRNA(s) is/are prepared by transcription of DNA(s) encoding the protein(s) comprising two or more cysteine residues.
[7] The method of any one of [1] to [6], wherein the translation system used in step (b) is a cell-free translation system.
[8] The method of any one of [1] to [7], wherein the determination whether the target substance(s) interact with the protein(s) is carried out by evaluating whether the two bind to each other through the affinity column chromatography method or the affinity bead method.
[9] The method of any one of [1] to [8], which further comprises the step of:
   identifying the protein(s) and/or the target substance(s) that have been determined to interact to each other.
[10] The method of any one of [2] and [4] to [9], wherein the mutagenesis of the DNA is achieved by a mutagenesis method such as the error-prone PCR.
[11] The method of any one of [1] to [10], wherein a protein with enhanced interactive force to the target substance(s) is obtained by repeating each of the steps several times.
[12] The method of any one of [1] to [11], wherein the mRNA(s) encodes/encode a protein/proteins comprising 8 to 500 amino acid residues.
[13] The method of any one of [1] to [12], wherein the mRNA(s) encodes/encode a protein/proteins comprising 10 to 200 amino acid residues.
[14] The method of any one of [1] to [13], wherein the mRNA(s) encodes/encode a protein/proteins comprising 2 to 10 cysteine residues.
[15] The method of any one of [1] to [14], wherein the mRNA(s) encodes/encode a protein/proteins in which the cysteine residues adjacent to each other are separated in the amino acid sequence with an interval of 2 to 20 residues.
[16] The method of any one of [1] to [15], wherein the mRNA(s) encodes/encode a protein/proteins in which the cysteine residues positioned to the most N-terminal side and the most C-terminal side are separated by 5 to 50 residues.
[17] The method of any one of [5] to [16], wherein the spacer comprises, as the major backbone, polynucleotide, polyethylene, polyethylene glycol, polystyrene, peptide nucleic acid, or a combination thereof.
[18] The method of any one of [5] to [17], wherein the spacer comprises a solid-phase immobilization site and wherein the mRNA-puromycin conjugate(s) is/are linked to a solid phase via the solid-phase immobilization site.
[19] The method of [18], wherein the solid phase is selected from the group consisting of:
   styrene bead, glass bead, agarose bead, Sepharose bead, magnetic bead, glass base, silicon base, plastic base, metallic base, glass container, plastic container, and membrane.
[20] The method of [18] or [19], wherein the solid-phase immobilization site in the spacer has a cleavable site, and wherein the method comprises the step of:
   folding the protein(s) on the solid phase and then cleaving the cleavable site.
**[21]** The method of [20], wherein the spacer is a DNA spacer and wherein the cleavable site is a restriction enzyme recognition site.
**[22]** A synthetic protein obtainable by the method of any one of [1] to [21], which has 8 to 500 amino acid residues, comprises 2 to 10 cysteine residues for forming disulfide bonds, and has an association constant to the target substance that changes due to oxidation and reduction.
[23] A synthetic protein which has 8 to 500 amino acid residues, comprises 2 to 10 cysteine residues for forming disulfide bonds, and has an association constant to the target substance that changes due to oxidation and reduction.

Hereinbelow, the present invention will be specifically described according to the embodiments thereof.

The present invention relates to methods for identifying useful proteins by synthesizing a protein randomly comprising a disulfide bond via the introduction of cysteine residues into the amino acid sequence of the protein, and analyzing the function of the protein.

A preferable embodiment of the present invention relates to methods for identifying useful proteins by synthesizing a protein comprising a disulfide bond via the introduction of cysteine residues into the amino acid sequence of the protein, and analyzing the function of the protein, wherein the method comprises the steps of (a) to (c):
(a) preparing one or more mRNAs encoding a protein/proteins that comprise at least two cysteine residues, and linking each of the prepared mRNAs with puromycin or a puromycin-like compound to obtain mRNA-puromycin conjugate(s) (hereinbelow, also referred to as "mRNA-PM conjugate(s)");
(b) contacting a translation system with the mRNA-PM conjugate(s) obtained in step (a) to synthesize the protein/proteins, and preparing mRNA-puromycin-protein conjugate(s) (hereinbelow, also referred to as "mRNA-PM-PRT conjugate(s)"); and
(c) contacting one or more target substances with the mRNA-PM-PRT conjugate(s) prepared in step (b), and determining whether the target substances interact with any one of the proteins within the mRNA-PM-PRT conjugate(s).

Herein, the phrase "introduction of cysteine residues into the amino acid sequence" preferably means that cysteine residues are introduced at random positions into the amino acid sequence (i.e., a natural protein-derived sequence or a synthetic sequence). However, in the present invention, the introduction of cysteine residues into the amino acid sequence is not restricted to random introduction and includes, for example, a process wherein cysteine residues are introduced to arrange the residues according to a certain rule or prearranged plan. The phrase a "protein comprising a disulfide bond" preferably means that the protein has a disulfide bond at random positions. However, similarly to the cysteine residues, it is not restricted to proteins that have disulfide bonds at random positions.

Each of the steps are specifically described below.

### 1. Step (a): Preparation of mRNA-puromycin conjugate(s)

### [Design of mRNA sequences]

For step (a), it is preferable to use artificially prepared mRNAs, due to the convenience of preparation and manipulation; however, wild-type mRNAs may also be used. The mRNAs used herein encode proteins comprising at least two or more cysteine residues to synthesize proteins comprising at least one or more disulfide bonds. The mRNAs encode proteins comprising preferably two to ten cysteine residues, more preferably four to ten cysteine residues. Herein, the term "protein" is intended to include peptides. To achieve formation of a disulfide bond by the cysteine residues in the protein that is synthesized through translation, at least two or more of the cysteine residues are separated preferably by two amino acid residues or more, and more preferably by three amino acid residues or more. In addition, it is preferred that the cysteine residues be dispersedly distributed to some extent in the amino acid sequence. Thus, sequences comprising evenly distributed cysteine residues are preferred over sequences with cysteine residues unevenly distributed to the N-terminal or C-terminal side. Furthermore, the mRNAs preferably encode proteins in which the cysteine residues positioned to the most N-terminal side and the most C-terminal side are separated by preferably 5 to 50 residues, more preferably 10 to 50 residues.

The mRNAs to be used in the present invention include those encoding proteins having preferably 10 to 200 amino acid residues, more preferably 10 to 100 amino acid residues, even more preferably 15 to 50 amino acid residues, and still more preferably 20 to 40 amino acid residues; nevertheless, the mRNAs are not limited thereto. This preference arises from the fact that extremely long mRNAs are difficult to prepare, and proteins synthesized from extremely short mRNAs are difficult to form proper tertiary structures.

Not all of the 20 types of amino acid residues have to be included in the protein.

The mRNAs to be used in the present invention will be appropriately designed, taking into account the above-described requirements. Such sequences that meet the above-described requirements can be readily designed by human power (of narrow sense) or with the help of a simple computer program. In the present invention, for example, when the target is a protein having 30 amino acid residues comprising 6 cysteines, mRNAs encoding proteins having the amino acid sequences as shown below (wherein, S1 to S6 represent cysteine residues; A1 to A30 represent amino acid residues other than the cysteine residue (e.g., glycine, alanine, valine, leucine, isoleucine, phenylalanine, tyrosine, tryptophan, proline, aspartic acid, glutamic acid, methionine, asparagine, glutamine, serine, threonine, lysine, arginine, or histidine)) are used, but are not limited thereto:
(1) A1-A2-S1-A4-A5-A6-S2-A8-A9-A10-S3-A12-A13-A14-S4-A16-A17-A18-S5-A20-A21-A22-S6-A24-A25-A26-A27-A28-A29-A30;
(2) A1-A2-A3-S1-A5-A6-A7-S2-A9-A10-A11-S3-A13-A14-A15-S4-A17-A18-A19-S5-A21-A22-A23-S6-A25-A26-A27-A28-A29-A30;
(3) A1-S1-A3-A4-A5-A6-S2-A8-A9-A10-A11-S3-A13-A14-A15-A16-S4-A18-A19-A20-A21-S5-A23-A24-A25-A26-S6-A28-A29-A30;
(4) A1-S1-A3-A4-A5-A6-A7-S2-A9-A10-A11-A12-A13-S3-A15-A16-A17-A18-A19-S4-A21-A22-A23-A24-S5-A26-A27-A28-S6-A30;
(5) A1-A2-S1-A4-A5-S2-A7-A8-A9-A10-A11-A12-A13-S3-A15-S4-A17-A18-A19-A20-A21-A22-A23-A24-S5-A26-A27-S6-A29-A30;
(6) A1-A2-A3-A4-A5-S1-A7-A8-S2-A10-A11-A12-A13-A14-A15-S3-A17-S4-A19-A20-A21-S5-A23-A24-A25-S6-A27-S6-A29-A30;
(7) A1-A2-A3-S1-A5-A6-A7-A8-A9-A10-A11-S2-A13-A14-A15-S3-A17-A18-A19-A20-A21-S4-A23-A24-A25-S5-A27-A28-S6-A30;
(8) A1-S1-A3-S2-A5-A6-A7-A8-A9-S3-A11-A12-S4-A14-A15-A16-A17-A18-A19-A20-A21-A22-A23-S5-A25-A26-A27-S6-A29-A30;
(9) A1-A2-A3-A4-A5-A6-S1-A8-A9-A10-S2-A12-A13-S3-A15-A16-A17-A18-A19-A20-S4-A22-A23-A24-A25-A26-S5-A28-S6-A30; or
(10) A1-S1-A3-A4-A5-A6-S2-A8-S3-A10-S4-A12-A13-A14-A15-A16-A17-S5-A19-A20-A21-A22-A23-A24-S6-A26-A27-A28-A29-A30.

A desired nucleic acid sequences may be designed based on an already known amino acid or nucleic acid sequence, by searching sequences that meet the above-described requirements. Information on such known amino acid sequences and nucleic acid sequences is available from known databases including, for example, Protein Data Bank (PDB), GenBank (National Center for Biotechnology Information), EMBL (European Bioinformatics Institute; EBI), and DDBJ (The National Institute of Genetics).

In addition to the disulfide bond, various factors, such as the hydrogen bond, ionic bond, and van der Waals forces, participate in the formation of the tertiary structure of a protein. Furthermore, amino acid sequences that form α-helix, β-sheet, zinc finger, leucine zipper, helix-turn-helix, helix-loop-helix, and such are known to those skilled in the art. Thus, taking these factors into consideration, as needed, more appropriate sequences can be designed using known techniques for computer-assisted molecular design. For example, when about three units of β-sheet structures are present in a protein, it is known that hydrogen bonds are formed between the β-sheet structures. Such information is useful for the formation of tertiary structures in addition to those formed due to the S-S bond by cysteine residues. The documents listed below provide useful information for designing such sequences:
(1) Nielsen, *KJ, et al.* J. Mol. Recognit., 13, 55-70, (2000);
(2) Norton, RS, & Pallaghy, Toxicon, 36, 1573-1583, (1998);
(3) Kim JII, *et al.,* J. Mol. Biol., 250, 659-671, (1995);
(4) Goldenberg, DP*., et al.,* Protein Science, 10, 538-550, (2001); and
(5) Miles, LA*., et al.,* J. Biol. Chem., 277, 43033-43040, (2002).

### [Preparation of mRNAs]

Once the amino acid sequences to be encoded by mRNAs of the present invention are designed as described above, the mRNAs can be synthesized according to known methods of nucleic acid synthesis (PerSeptive Biosystems, DNA/RNA Synthesis System, 1995). Alternatively, such mRNAs can be easily prepared by: synthesizing DNAs encoding proteins having the amino acid sequences designed as described above; and subjecting the DNAs to transcription reaction (Krupp G & Soll D, FEBS Lett., 212, 271-275, (1987)).

According to a preferred embodiment of the present invention, a DNA library encoding a preferred group of proteins may be prepared using a DNA synthesizer based on the above-described designing method, and mRNAs transcribed from the DNA library can be used for the present invention.

### [Puromycin and puromycin-like compounds]

An mRNA-puromycin (mRNA-PM) conjugate is typically prepared by linking the mRNA obtained as described above via a spacer at the 3'-terminus of the mRNA to puromycin or a puromycin-like compound. The puromycin and puromycin-like compound herein function as a hinge or junction linking the mRNA and translated protein when the mRNA-PM conjugate is subjected to a translation system to synthesize the protein. Specifically, it is known that *in vitro* virus virion wherein the mRNA is linked to the translated protein via puromycin is generated upon the contact of the translation system with the mRNA that is conjugated with puromycin via a spacer (see Nemoto *et al.,* FEBS Lett., 414, 405, (1997)). Puromycin is a compound with a 3'-terminus whose backbone chemical structure is similar to that of aminoacyl tRNA, and is represented by formula (I) as follows: 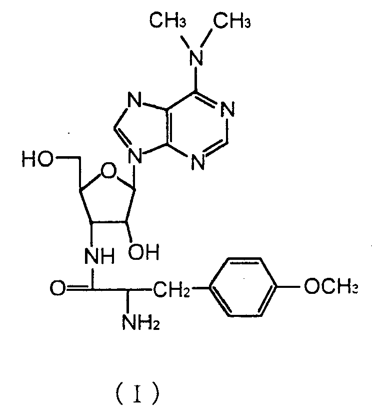 Puromycin is capable of binding to the C-termini of proteins synthesized in the translation system. Herein, the phrase "puromycin-like compound" refers to a compound with a 3'-terminus whose backbone chemical structure is similar to that of the aminoacyl tRNA, and which compound is capable of binding to the C-termini of proteins that is synthesized in translation system.

Examples of puromycin-like compounds include 3'-N-aminoacylpuromycin aminonucleosides (PANS-amino acids), such as PANS-Gly wherein the amino acid portion is glycine, PANS-Val wherein the amino acid portion is valine, PANS-Ala wherein the amino acid portion is alanine, and other PANS-amino acid compounds wherein the amino acid portion is any of the amino acids. Furthermore, 3'-N-aminoacyladenosine aminonucleosides (AANS-amino acids) that is formed by dehydrative condensation reaction of the amino group of 3'-aminoadenosine and the carboxyl group of an amino acid can also be used; these include compounds such as AANS-Gly wherein the amino acid portion is glycine, AANS-Val wherein the amino acid portion is valine, AANS-Ala wherein the amino acid portion is alanine, and other AANS-amino acid compounds wherein the amino acid portion is any of the amino acids. Nucleosides and nucleosides linked with amino acids via an ester bond may also be used. In addition to the puromycin described above, preferably used puromycin-like compounds include ribocytidyl puromycin (rCpPur), deoxycytidyl puromycin (dCpPur), deoxyuridyl puromycin (dUpPur), and the like. Their chemical structures are shown below.

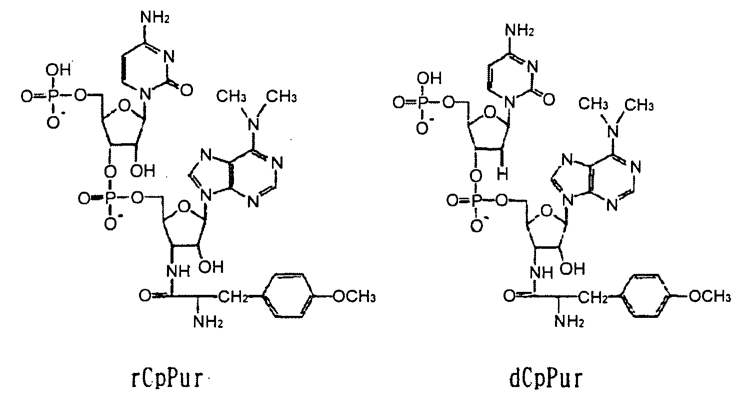

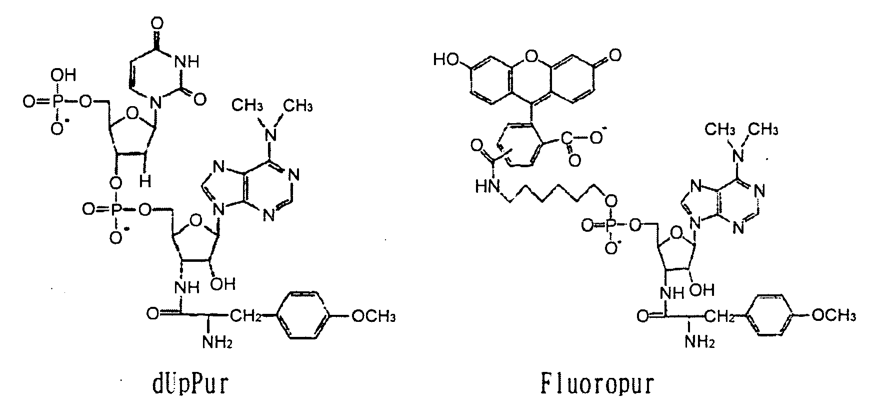

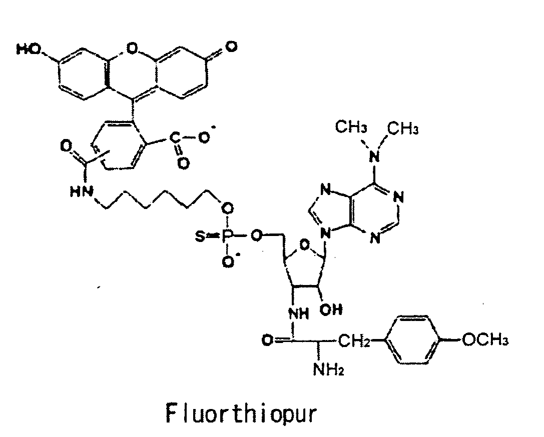

### [Spacers]

In an mRNA-PM conjugate of the present invention, the mRNA is linked with puromycin or a puromycin-like compound (hereinafter simply referred to as "puromycin") via a spacer. The conjugate is immobilized onto a solid phase, preferably via a solid-phase immobilization site designed within the spacer. The spacer is mainly used for efficient incorporation of puromycin into the site so-called "A site" of the ribosome. Thus, the spacer is not particularly limited, so long as it has these properties; however, spacers that are flexible and hydrophilic, and that have a simple backbone structure with less side chains are preferred. Specifically preferred spacers for use herein include, but are not limited to, polynucleotides (including DNAs); polyalkylenes, such as polyethylene; polyalkylene glycols, such as polyethylene glycol; peptide nucleic acids (PNAs); linear substances, such as polystyrene; and those comprising a combination thereof as the major backbone. When the above-described linear substances are used in combination, they can be chemically linked together as needed via adequate linkage groups (-NH-, -CO-, -O-, -NHCO-, -CONH-, -NHNH-, -(CH₂)ₙ₋[wherein 'n' represents, for example, 1 to 10, preferably 1 to 3], -S-, -SO-, etc.).

The mRNA can be directly or indirectly, chemically or physically, linked with a spacer through known methods. For example, when DNA is used as the spacer, the mRNA can be linked with the DNA spacer by designing a sequence complementary to the end of the DNA spacer at the 3'-terminus of the mRNA. Furthermore, to link a spacer with puromycin, generally, known chemical methods are used.

When cleavage of the complex, mRNA-PM-protein (mRNA-PM-PRT), from the solid phase is required after protein synthesis, it is preferred to design a cleavable site within the spacer. When DNA is used as a portion of the spacer, a restriction enzyme recognition site may be used as the cleavable site within the DNA strand. When such a spacer with a restriction enzyme recognition site is used, an mRNA-PM-protein complex can be cleaved from the solid phase by adding a restriction enzyme (for example, *Alu*I*, Bam*HI, *Eco*RI, *Hin*dII, *Hin*d*III,* or *Pvu*II) thereto at preferred time, for example, after protein synthesis. Combinations of restriction enzyme recognition sites and enzymes that cleave the sites are known in the art (see, for example, New England BioLabs 2000/O1 catalog & technical reference).

The mRNA-PM conjugate of the present invention can be labeled by binding a labeling substance, as required. Such a labeling substance is appropriately selected from fluorescent substances, radioactive labeling substances, and so on. Various types of fluorescent dyes can be used, that have a free functional group (for example, a carboxyl group that can be converted into active ester; a hydroxyl group that can be converted into phosphoramidite; or an amino group) and that can be linked to a spacer, puromycin, or a puromycin-like compound. Suitable labeling substance include, for example, fluorescent substances such as fluorescein isothiocyanate (FITC), phycobiliprotein, rare earth metal chelates, dansyl chloride, and tetramethylrhodamine isothiocyanate; and radioisotopes such as ³H, ¹⁴C, ¹²⁵I, and ¹³¹I.

### [Solid phases]

The solid phase onto which an mRNA-PM conjugate is immobilized is not particularly limited and may be appropriately selected depending on the application purpose of the conjugate. The solid phase to be used in the present invention include those used as carriers onto which biomolecules are immobilized, exemplified by: beads, such as styrene beads, glass beads, agarose beads, Sepharose beads, and magnetic beads; bases, such as a glass base, silicon (silica) base, plastic base, and metal base (for example, gold leaf base); containers, such as a glass container and plastic container; and membranes made of, for example, nitrocellulose or polyvinylidene fluoride (PVDF).

Means for immobilizing an mRNA-PM conjugate of the present invention onto a solid phase are not particularly limited, so long as the immobilization does not inhibit the translation of the mRNA during the contact with a translation system. Typically, a solid-phase immobilization site is designed within the spacer linking the mRNA and PM, and the mRNA-PM conjugate is immobilized onto the solid phase *via* a "solid-phase immobilization site recognition site" linked to the solid phase. The solid-phase immobilization site is not particularly limited, so long as it allows the linkage between the mRNA-PM conjugate and a preferred solid phase. For example, molecules (for example, ligands and antibodies) specifically binding to particular polypeptides may be used as the solid-phase immobilization site. In this case, as the solid-phase immobilization site recognition site, the particular polypeptide that binds to the molecule is immobilized to the surface of the solid phase. Combinations of the solid-phase immobilization site and the solid-phase immobilization site recognition site include, for example, a biotin-binding protein, such as avidin or streptavidin, and biotin; a maltose-binding protein and maltose; a G protein and guanine nucleotide; a polyhistidine peptide and metal ion, such as nickel or cobalt; glutathione-S-transferase and glutathione; a DNA-binding protein and DNA; an antibody and antigen molecule (epitope); calmodulin and a calmodulin-binding peptide; an ATP-binding protein and ATP; and various combinations of receptor proteins and ligands thereof, such as an estradiol receptor protein and estradiol. Among them, preferred combinations of the solid-phase immobilization site and the solid-phase immobilization site recognition site are biotin-binding protein, such as avidin or streptavidin, and biotin; a maltose-binding protein and maltose; a polyhistidine peptide and metal ion, such as nickel or cobalt; glutathione-S-transferase and glutathione; and an antibody and antigen molecule (epitope); most preferred is the combination of streptavidin and biotin.

The above-described proteins can be immobilized on the surface of a solid phase by known methods, which include, for example, methods utilizing tannic acid, formalin, glutaraldehyde, pyruvic aldehyde, bisdiazotized bendizone, toluene-2,4-di-isocyanate, carboxyl group, hydroxyl group, or amino group (see P. M. Abdella, P. K. Smith, G P. Royer, A New Cleavable Reagent for Cross-Linking and Reversible Immobilization of Proteins, Biochem. Biophys. Res. Commun., 87, 734 (1979); etc.).

The above described combinations can also be used by reversing the sites of solid-phase immobilization and solid-phase immobilization site recognition site. Two substances having affinity for each other were used in the above-described immobilization method. However, when the solid phase is plastic material such as styrene beads or base, if required, a portion of the spacer may be directly linked to the solid phase via covalent bond using known techniques (see, LiquiChip Applications Handbook, Qiagen; etc.). According to the present invention, the immobilization means are not limited to the above-described methods, and any immobilization means known to those skilled in the art may be used.

### 2. Step (b): Preparation of mRNA-PM-PRT conjugate(s)

In Step (b), mRNA-PM-PRT conjugate(s) is/are prepared by synthesizing proteins through the contact of the mRNA-PM conjugates with a translation system. Examples of translation system that can be used in this step include cell-free translation systems, viable cell translation systems, and so on. Cell-free translation systems constituted by prokaryotic or eukaryotic extract, for example, E. *coli* extract, rabbit reticulocyte extract, and wheat germ extract, can be used as the cell-free translation systems (see, Lamfrom H, Grunberg-Manago M. "Ambiguities of translation of poly U in the rabbit reticulocyte system." Biochem Biophys Res Commun., 27(1), 1-6, (1967)). Prokaryotic or eukaryotic cells, such as E. *coli* cells, can be used as the viable cell translation systems. In the present invention, the cell-free systems are preferred, due to ease of handling.

If required, the obtained mRNA-PM-PRT conjugate(s) may be subjected to oxidizing and reducing treatment to form a disulfide bond (S-S bond). For example, the obtained mRNA-PM-PRT conjugate(s) may be reduced by reaction in the presence of a reducing agent such as dithiothreitol (DTT), at 0°C to 35°C for 0.5 hr to 3 hr. Then, the mRNA-PM-PRT conjugate(s) treated as described above is/are then reacted in the presence of an oxidizing agent such as o-iodosobenzoic acid, in an appropriate buffer (for example, 50 mM phosphate buffer (pH7)) at 0°C to 35°C for 5 min to 1 hr. Washing can be carried out after each treatment, if required, using an appropriate washing buffer (for example, washing buffer (Tris-HCl (pH8.0)/100 mM NaCI)).

According to a preferred embodiment of the present invention, the above-described solid-phase immobilization site in the spacer has a cleavable site, and in Step (b), after reliable folding of the proteins on the solid phase, the mRNA-puromycin-protein conjugate(s) is/are cleaved from the solid phase by cleaving the cleavable site. Then, the cleaved mRNA-puromycin-protein conjugate(s) is/are subjected to Step (c). Through such manipulation, screening and functional analysis can be performed after achieving secure protein folding.

In the present invention, the folding of a protein can be achieved through disulfide bond formation, preferably, by oxidizing the SH groups of the cysteine residues contained in the protein. The proteins formed through such procedure as described above have a stable tertiary structure. In general, the function of a protein depends on its tertiary structure. Therefore, the method of the present invention enables efficient screening of functional proteins depending on their tertiary structures.

According to a preferred embodiment of the present invention, the folding of a protein is achieved under a condition wherein the protein is immobilized on a solid phase. An S-S bond can be efficiently formed by subjecting the solid-phase immobilized conjugate of the present invention to oxidization reaction.

### 3 Step (c): Assay for interactions between protein and target substance

In Step (c), one or more target substances are contacted with the mRNA-PM-PRT conjugate(s) prepared in Step (b). As used herein, the phrase "target substance" refers to a substance that is used for determining whether it interacts with a protein synthesized in the present invention, and specifically includes proteins, nucleic acids, sugar chains, and low-molecular-weight compounds.

The protein of the present invention is not particularly limited, and may be a full-length protein or a partial peptide comprising a site with binding activity. Furthermore, it may be a protein with known or unknown amino acid sequence and function. As the target molecules, synthesized peptide chains, proteins purified from living bodies, purified proteins translated from a cDNA library or such using an appropriate translation system, and such can be used. The synthesized peptide chains may be glycoproteins, in which sugar chains are bound to the peptide chains. Among them, the purified proteins with known amino acid sequences and those translated from a cDNA library or such via an appropriate method are preferred.

The nucleic acids are not particularly limited and both DNAs and RNAs can be used. Furthermore, nucleic acids with known or unknown nucleotide sequences or functions may be used. Preferred nucleic acids to be used include those with known nucleotide sequences and functions as having the binding ability to a protein, and those isolated from a genomic library by cleavage using a restriction enzyme or such.

The sugar chains are not particularly limited, and may include those with known or unknown sequences or functions. Previously isolated and analyzed sugar chains with known sequences or functions are preferred.

The low-molecular-weight compounds are not particularly limited, and those with unknown functions and those known to have the ability to bind to a protein can be used.

The term "interaction" between these target substances and a protein generally refers to an action based on the intermolecular force caused by at least one of the covalent bond, hydrophobic bond, hydrogen bond, van der Waals attraction, and bonds by electrostatic force between the protein and a target molecule. However, this term should be understood in the broadest sense, and in any case should not be limited. The phrase "covalent bond" includes coordinate bonds and dipole bonds. The phrase "bonds by electrostatic force" includes electrostatic repulsion in addition to electrostatic bonds. The binding reaction, synthesis reaction, and decomposition reaction caused by the above-described action are also included within the term "interaction". Specific examples of interactions include:
- association and dissociation between an antigen and an antibody;
- association and dissociation between a protein receptor and a ligand;
- association and dissociation between an adhesion molecule and its partner molecule;
- association and dissociation between an enzyme and a substrate;
- association and dissociation between a nucleic acid and a protein binding thereto;
- association and dissociation between proteins involved in signal transduction;
- association and dissociation between a glycoprotein and a protein; and
- association and dissociation between a sugar chain and a protein. Thus, the interactive force can be evaluated by determining the degree of binding (association constant) and/or degree of dissociation (dissociation constant) between the two substances (LeTilly V. & Royer CA, Biochemistry, 32, 7753-7758, (1993)). Herein, the phrase "association constant to a target substance that changes due to oxidation and reduction" typically refers to changes of one or more order of magnitude (for example, from 10⁹ to 10⁸).

The target substances used herein may be used after labeling with a labeling substance, if required. The labeling can be carried out by appropriately linking the labeling substance. Such labeling substances may be appropriately selected from fluorescent substances, radioactive labeling substances, and such. It is possible to use, as the fluorescent substances, various types of fluorescent dyes that have a free-functional group (for example, carboxyl group that can be converted into active ester, hydroxyl group that can be converted into phosphoamide, or amino group) and that can be linked with the target substance. Suitable labeling substances include, for example, fluorescent substances such as fluorescein isothiocyanate, phycobiliprotein, rare earth metal chelates, dansyl chloride, tetramethylrhodamine isothiocyanate, and Cy3; and radioisotopes such as ³H, ¹⁴C, ¹²⁵I, and ¹³¹I. Among these labeling substances, those adapted for the method of measuring or analyzing the change in the signal generated based on the interaction between the target substance and the immobilized protein are appropriately used. The binding of the above-described labeling substance to the target substance can be carried out according to known methods.

In Step (c), it is determined whether the protein interacts with the target substance. The presence of interaction between the protein and the target substance is determined by measuring and detecting the alteration in the signal generated based on the interaction between the two molecules. Such measurement methods include, for example, the surface plasmon resonance method (Cullen, D. C., *et al.,* Biosensors, 3(4), 211-225, (1987-88)), the evanescent field molecular imaging (Funatsu, T., *et al.,* Nature, 374, 555-559, (1995)), the fluorescence imaging analysis, the enzyme-linked immunosorbent assay (ELISA) (Crowther, J. R., Methods in Molecular Biology, 42, (1995)), the fluorescence depolarization method (Perran, J., *et al.,* J. Phys. Rad., 1, 390-401, (1926)), and the fluorescence correlation spectroscopy (FCS) (Eigen, M., *et al.,* Proc. Natl. Acad. Sci. USA, 91, 5740-5747, (1994)).

The interaction between the two molecules can be evaluated by simply determining whether or not the two molecules bind to each other. Such a binding experiment can be achieved by methods (for example, affinity chromatography) that utilize the specific affinity of the two molecules. Specifically, an mRNA-PM-PRT conjugate that binds to a target substance in a sample can be separated by: (1) immobilizing (immobilization via physical adsorption, polymerization by cross-linking, matrix embedding, non-covalent bond, and so on) the target substance onto an insoluble carrier (for example, carriers such as bead, filter, and membrane) such as cellulose carrier, agarose carrier, polyacrylamide carrier, dextran carrier, polystyrene carrier, polyvinyl alcohol carrier, poly-amino acid carrier, or porous silica carrier through conventional methods; (2) filling the insoluble carrier into a column made of glass, plastic, or stainless; and (3) eluting the sample through the column (for example, cylindrical column). This procedure also allows to select only the required proteins by excluding proteins that did not bind to the target substance from the screening process. Alternatively, the binding experiment can be carried out using an affinity bead method that utilizes the specific affinity of the two molecules (Ogata Y, *et al.,* Anal Chem., 74, 4702-4708, (2002)). Specifically, a magnetic bead having streptavidin or a target molecule immobilized to its surface via an amino group can be used. An mRNA-PM-PRT conjugate that binds to a biotinylated target molecule can be isolated by contacting it with a streptavidin bead and collecting the streptavidin magnetic bead with a magnet. Alternatively, the mRNA-PM-PRT conjugate can be isolated by contacting it with the magnetic bead on whose surface the target molecule is immobilized, and collecting the magnetic bead with a magnet. Furthermore, when an agarose bead or such is used in place of the magnetic bead, the isolation can be carried out by collecting only the bead through precipitation, using a centrifuge instead of a magnet.

In the methods of the present invention, if required, the protein and/or target substance of the protein-target substance complex, which have been determined to interact with each other in Step (c) are identified. The identification of the protein can be carried out with an conventional amino acid sequencer; or by analyzing the nucleotide sequence of DNA that is obtained by reverse transcription of mRNA bound to the protein. The identification of the target substance can be achieved by NMR, IR, and various types of mass spectrometries.

### 4. Step (d): Preparation of altered proteins

According to the present invention, a protein with enhanced interactive force to a target substance can be obtained by preferably conducting, after Step (c), an additional Step (d), which comprises: (1) preparing DNA(s) via reverse transcription of the mRNA(s) of the mRNA-puromycin-protein conjugate(s) comprising a protein that interacts with the above-described target substance; (2) preparing mRNAs having altered sequences from the DNA(s) by mutagenesis; (3) subjecting the mRNAs to Step (a); and then (4) performing the treatment of Steps (b), and (c) as described above. The mutagenesis of the DNA can be achieved by known methods, such as the error-prone PCR method (Gram H*, et al.,* Proc. Natl. Acad. Sci. USA., 89, 3576-80, (1992)) and the DNA shuffling method (Stemmer WP, Nature, 370, 389-391, (1994)). In the present invention, the error-prone PCR method, which allows low frequency random mutagenesis of DNA, is preferred. A kit to perform the error-prone PCR method is commercially available under the name of "GeneMorph™ PCT Mutagenesis Kit" from Stratagene. When a protein that interacts somewhat with a proper target substance has been isolated via the above-described Steps (a) to (c), through the method as described above, a protein with stronger activity can be obtained by randomly altering the amino acid sequence of the isolated protein. Thus, a protein with more enhanced interactive force to a target substance can be obtained by repeating Steps (a) to (d) several times.

Furthermore, a protein having much more enhanced activity can be obtained when plural proteins that interact with a particular target substance have been obtained by repeating such steps several times (for example, twice to ten times, preferably four to eight times), by designing a protein having a novel amino acid sequence based on the consensus sequence information and such of the amino acid sequences of the plural proteins, and subjecting the newly designed protein to Step (a) described above.

Thus obtained proteins can be synthesized according to known organic synthesis methods (see, N. Izumiya, *et al.,* "Fundamental principles and Experiments of Peptide Synthesis", Maruzen Co. Ltd. (1975); etc.). Proteins that are obtainable through such screening methods can be used for various pharmaceutical purposes, as physiologically active substances that modulate the physiological function of a target substance.

In Step (c), the mRNA-puromycin-protein conjugate(s) is/are contacted with a target substance. However, in the present invention, DNA(s) is/are prepared by reverse transcription of the mRNA(s) of the mRNA-puromycin-protein conjugate(s) to prepare DNA-puromycin-protein conjugate(s) which then may be contacted with the target substance.

Generally, DNA molecules are known to be more stable than RNA molecules. In addition, DNA molecules are more resistant to alkali than RNA molecules. Thus, a DNA-puromycin-protein conjugate is more convenient for achieving chemical reactions, for example, when used in cross-linking reactions.

Moreover, in the present invention, the DNA/mRNA-puromycin conjugate(s) prepared by the above-described steps of the present invention may be stored and then appropriately contacted with a target molecule. It is known that DNA molecules generally are stable even after repeating freezing and thawing, while RNA molecules are generally easily degraded by repeating freezing and thawing. It is also known that RNA molecules are more influenced by non-specific interaction than DNA molecules. Thus, in the present invention, the DNA-puromycin-protein conjugate(s) is preferred as the conjugate to be contacted with the target molecule.

Specifically, another preferable embodiment of the present invention relates to methods of screening for useful proteins by synthesizing a protein comprising a disulfide bond via the introduction of cysteine residues into the amino acid sequence of the protein, and analyzing the function of the protein, wherein the method comprises the steps of (a) to (d):
(a) preparing one or more mRNAs encoding a protein/proteins that comprise at least two cysteine residues, and linking each of the prepared mRNAs with puromycin or a puromycin-like compound to obtain mRNA-puromycin conjugate(s);
(b) contacting a translation system with the mRNA-puromycin conjugate(s) obtained in step (a) to synthesize a protein/proteins, and preparing mRNA-puromycin-protein conjugate(s);
(c) preparing DNA(s) via reverse transcription of the mRNA(s) of the mRNA-puromycin-protein conjugate(s) obtained in step (b), and preparing DNA-puromycin-protein conjugate(s); and
(d) contacting one or more target substances with the DNA-puromycin-protein conjugate(s) prepared in step (c), and determining whether the target substances interact with any one of the proteins of the DNA-puromycin-protein conjugate(s).

All prior art documents cited herein are incorporated herein by reference.

### Brief Description of the Drawings

Fig. 1 is a schematic illustration showing an embodiment of the screening method of the present invention.
Fig. 2 is a schematic illustration of an mRNA that is covalently bonded with a puromycin-attached spacer DNA.
Fig. 3 is a schematic illustration showing a PM-attached spacer DNA. "P" represents puromycin; "F" represents FITC; and "B" represents biotin. "A", "T", "C", and "G" represent the sequence of DNA; and "a", "u", "g", and "c" represent the sequence of RNA. The boxed portion indicates the *Pvu*II restriction site. The 3'-terminus of the RNA and the 5'-terminus of the DNA were synthesized so as to be ligated to each other at the portion indicated as "T4 RNA Ligase".
Fig. 4 is a illustration showing the sequence constitution of a full-length DNA library.

### Best Mode for Carrying Out the Invention

The present invention is specifically described below with reference to Examples; however, it is not to be construed as being limited thereto.

### Experimental Example 1

### 1. Synthesis of spacer BioLoop-Puro (hereinafter abbreviated as "PM-attached spacer DNA")

10 nmol of Puro-F-S [sequence: 5'-(S)-TC(F)-(Spec18)-(Spec18)-(Spec18)-(Spec18)-CC-(Puro)-3'; purchased from BEX] was dissolved in 100 µl of 50 mM phosphate buffer (pH7.0), and 1 µl of 100 mM Tris(2-carboxyethyl)phosphine hydrochloride (TCEP) was added (at a final concentration of 1 mM) thereto. The mixture was allowed to stand at room temperature for 6 hrs to reduce the thiol groups of Puro-F-S. Immediately before the cross-linking reaction, TCEP was removed using NAP5 (Amersham; 17-0853-02) that had been equilibrated with 50 mM phosphate buffer (pH7.0). In the sequence of Puro-F-S, "(S)" represents 5'-thiol-modifier C6; and "(Puro)" represents puromycin CPG. "Spec 18" represents the spacer (18-O-Dimethoxytritylhexaethyleneglycol, 1-[(2-cyanoethyl)-(N,N-diisopropyl)]-phosphoramidite) which is manufactured by Glen Research Search Co. and has the following chemical structure:

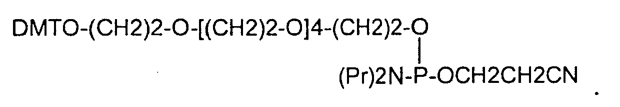

20 µl of 500 pmol/µl biotin-loop [(56 mer) sequence: 5'-CCCGG TGCAG CTGTT TCATC (T-B) CGGAAACAG CTGCA CCCCC CGCCG CCCCC CG(T)CCT-3' (SEQ ID NO: 1); purchased from BEX; wherein "(T)" represents amino-modifier C6 dT, and "(T-B)" represents biotin-dT (the *Pvu*II restriction site is underlined)] and 20 µl of 100 mM cross-linking agent EMCS (344-05051; 6-Maleimidohexanoic acid N-hydroxysuccinide ester; Dojindo Co.) were added to 100 µl of 0.2 M phosphate buffer (pH7.0). The resulting mixture was stirred well and then left to stand at 37°C for 30 min to remove any unreacted EMCS. The precipitate was dried under reduced pressure and dissolved in 10 µl of 0.2 M phosphate buffer (pH7.0). Then, the reduced Puro-F-S (up to 10 nmol) described above was added thereto and was allowed to stand overnight at 4°C. TCEP was added at a final concentration of 4 mM to the sample and allowed to stand at room temperature for 15 min. Unreacted Puro-F-S was removed by ethanol precipitation and to remove unreacted Biotin-loops, HPLC purification was preformed under the following condition:
Column: nacalai tesque CSOMOSIL 37918-31 10 x 250 mm C18-AR-300 (Waters)
Buffer A: 0.1 M TEAA
Buffer B: 80% acetonitrile (diluted with ultra pure water)
Flow rate: 0.5 ml/min (B%: 15% to 35%, 33 min).

HPLC fractions were analyzed using 18% acrylamide gel (8 M urea, 62°C). The fractions of interest were dried under reduced pressure, and dissolved in DEPC-treated water to adjust the concentration to 10 pmol/µl.

### 2. Preparation of template DNAs for antibody-like peptides

To form at least two or more S-S bonds in each molecule of the antibody-like peptides, four or more cysteine residues were included in one peptide. Thus, sequences comprising four or more codons corresponding to cysteine, UGU or UGC, were produced as template DNAs.

In this Example, a DNA library as described below (SEQ ID NO: 4 and 5; sequences complementary to each other) that encodes proteins having the amino acid sequence as shown below (SEQ ID NO: 2 or 3) was synthesized by the solid phase synthesis method using a DNA synthesizer (Applied Biosystems; 3400).

SEQ ID NO: 2
Xaa-Xaa-Xaa-Xaa-Cys-Xaa-Xaa-Xaa-Cys-Xaa-Xaa-Xaa-Cys-Xaa-Xaa-Xaa-Xaa-Cys-Xaa-Xa
a-Xaa-Xaa-Cys-Xaa-Xaa-Xaa-Xaa-Xaa-Xaa-Xaa-Xaa-Cys-Xaa-Xaa-Xaa-Xaa

SEQ ID NO: 3
Xaa-Xaa-Cys-Xaa-Xaa-Xaa-Xaa-Xaa-Xaa-Xaa-Xaa-Xaa-Cys-Xaa-Xaa-Cys-Xaa-Xaa-Xaa-Xa
a-Xaa-Cys-Xaa-Xaa-Xaa-Xaa-Xaa-Cys-Xaa-Xaa-Xaa-Cys-Xaa-Xaa-Xaa-Xaa

SEQ ID NO: 4
TTTCCCCGCCCCCCGTCCTGCTTCCGCCGTGATGATGATGATGATGGCCTCCGCTTG
GAGGGCCGGAGGGNNNNNNNNNNNNACANNNNNNNNNNNNNNNNNNNNNNNNNN
ACANNNNNNNNNNNNNACANNNNNNNNNNNNNACANNNNNNNNNNACANNNNNNN
NNACANNNNNNNNNNNNNCATGGTGGCTTGTAGTTGTAGAATGTAAAATGTAATG

SEQ ID NO: 5
CATGGTGGCTTGTAGTTGTAGAATGTAAAATGTAATGNNNNNTGTNNNNNNNNN
NNNNNNNNNNNNNNNNNNNTGTNNNNNNTGTNNNNNNNNNNNNNNNNNTGTNNNNNN
NNNNNNNNNNTGTNNNNNNNNNNNTGTNNNNNNNNNNNNNNCCCTCCGGCCCTCCAAGC
GGAGGCCATCATCATCATCATCACGGCGGAAGCAGGACGGGGGCGGGGAAA

Using the DNA library as a template and DNA primers (CATTACATTTTACATTCTACAACTACAAGCCACCATG (SEQ ID NO: 6); and TTTCCCCGCCCCCCGTCCT (SEQ ID NO: 7)), PCR was carried out (under the condition of: thermal denaturation at 95°C for 2 min, followed by 30 cycles of thermal denaturation at 95°C for 30 sec, annealing at 69°C for 15 sec, and extension at 72°C for 45 sec). The amplification product was purified by ethanol precipitation. Then, a DNA comprising the promoter sequence and non-coding region sequence of T7 (hereinafter abbreviated as T7Ω) (GATCCCGCGAAATTAATACGACTCACTATAGGGGAAGTATTTTTACAACAATTACCA ACAACAACAACAAACAACAACAACATTACATTTTACATTCTACAACTACAAGCCAC CATG (SEQ ID NO: 8)) was linked thereto by PCR (under the condition of: thermal denaturation at 95°C for 2 min, followed by 30 cycles of thermal denaturation at 95°C for 30 sec, annealing at 60°C for 15 sec, and extension at 72°C for 50 sec) without any primers. The resulting products were purified by phenol extraction and ethanol precipitation. Eventually, a template DNA was synthesized comprising, on the 5'-side, T7, Cap, omega sequence, and Kozak sequence and, on the 3'-side, a tag sequence partially complementary to spacer DNA (5'- AGGACGGGGGGCGGGGAAA (SEQ ID NO: 9); the region complementary to the spacer sequence is underlined) in addition to a x6 histidine tag.

### 3. Transcription

Using the template DNA synthesized in above-described Step 2 as a template, *in vitro* transcription was conducted according to the protocol of Promega Co. Specifically, m7G Cap Analog (Promega Co.) was added at a final concentration of 320 µM to 8 µg of the DNA, and incubated at 37°C for 1 hr. After DNase and phenol-chloroform treatments, the product was purified by DS Primer Remover (Edge Biosystems) and quantified.

### 4. Conjugation of mRNAs and PM-attached spacer DNAs

300 pmol of PM-attached spacer DNA, 6 µl of 10x ligation buffer (TaKaRa), and 2.5 µl of DMSO were added to 300 pmol of mRNA comprising 3'-terminal tag sequence and 5' cap, and DEPC-treated water was then added to a final volume of 55 µl. Annealing was performed through the incubation in a hot water bath, by changing the temperature from 85°C to 35°C in 20 min. 15 units of T4 polynucleotide kinase (TaKaRa; 2.5 µl) and 100 units of T4 RNA ligase (TaKaRa; 2.5 µl) were added to the mixture, and incubated at 25°C for 45 min. The resulting sample was treated with RNeasy Mini Kit (Qiagen, 74104), and then further purified by DS Primer Remover.

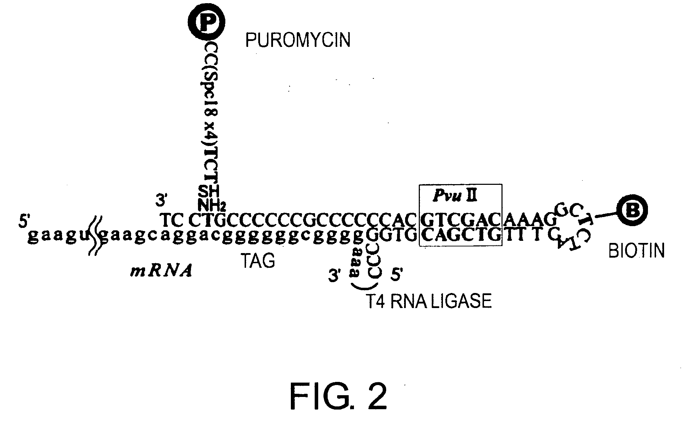

A schematic illustration of the mRNA wherein the PM-attached spacer DNA has been covalently bonded is shown in Fig. 2. In this figure, "P" represents puromycin; "B" represents biotin; and ATCGu represent DNA and RNA sequences. Capital letters indicate the DNA sequence comprising a *Pvu*II restriction site (boxed portion); and small letters indicate the mRNA sequence whose region complementary to the 3'-terminal DNA sequence is linked to the Tag sequence. The 3'-terminus of the RNA and the 5'-terminus of the DNA are ligated at the portion indicated "T4 RNA ligase".

### 5. Immobilization of PM-attached spacer DNA-mRNA complex onto beads

The complex of mRNA and PM-attached spacer DNA synthesized as described above was immobilized onto avidin beads (MAGNOTEX-SA, TaKaRa, 9088) with a diameter of 2.3 µm ± 0.3 µm following the appended protocol as described below.

60 µl of avidin beads were washed twice with 200 µl of 1x Binding Buffer (appended) by precipitating the avidin beads using a magnetic stand and exchanging the supernatant. After washing, 48 pmol of the complex of mRNA and PM-attached spacer DNA synthesized in Step 2-2 was added to the precipitated beads, 1x Binding Buffer (appended) was added thereto to a total volume of 120 µl, and was allowed to stand for 10 min at room temperature. Then, the beads were similarly washed with 200 µl of 1x Binding Buffer (appended) to remove complexes ofmRNA and PM-attached spacer DNA that failed to immobilize onto the beads. Furthermore, the beads were similarly washed by adding 10 µl of 20x Translation Mix (Ambion) and 190 µl of DEPC-treated water.

### 6. Translation

The above-described beads were precipitated onto the magnetic stand, and 300 µl of cell-free translation system (Retic Lysate IVT Kit; Ambion Co.; 1200) was added thereto to perform translation reaction at 30°C for 15 min. Then, MgCl₂ and KCl were added to final concentrations of 63 mM and 750 mM, respectively, and allowed to stand at 37°C for 1.5 hr. The sample was gently stirred about every one hour. The beads were precipitated as described above, and washed twice with 200 µl of 1x Binding Buffer (appended) containing 20 units of SUPERaseIn (Ambion Co.; 2694).

### 7. Reverse transcription

After washing, reverse transcription was performed to the beads precipitated similarly as described above at 42°C for 10 min, using reverse transcriptase M-MLV (TaKaRa; 2640A) at a scale of 80-µl following the protocol appended by TaKaRa BIOMEDICALS. Then, the beads were washed with 200 µl of 1x Binding Buffer (attached) similarly as described above.

### 8. Oxidizing and reducing treatment

The peptide translated in Step 6 and immobilized onto the beads in Step 7 was reduced through a reaction with 100 mM dithiothreitol (DTT) at room temperature for 1 hr. Then, the beads were washed twice with 200 µl of Washing Buffer (Tris-HCl (pH8.0), 100 mM NaCl). Next, to oxidize the peptide, the beads were reacted with 50 mM o-iodosobenzoic acid in 50 mM phosphate buffer (pH7) at room temperature for 10 min, and then washed twice with 200 µl of Washing Buffer (Tris-HCl (pH8.0), 100 mM NaCl).

### 9. Recovery of DNA-protein from beads

The DNA-protein was separated from the beads by incubating the precipitated beads with 24 units of restriction enzyme *Pvu*II (TaKaRa) at 40-µl scale at 37°C for 1 hr according to the appended protocol. In this step, BSA was added at a final concentration of 0.1 mg/ml to particularly prevent non-specific adsorption of the DNA-protein to the beads. Then, the beads were precipitated similarly as described above, and the supernatant was transferred into a fresh tube.

### 10. Purification of His tag

According to the protocol of Qiagen Co., the sample was diluted with an equal volume of lysis buffer (50 mM NaH₂PO₄, 300 mM NaCl, 10 mM imidazole, 0.05% Tween20 (pH8.0)), 20 µl of Ni-NTA beads (Ni-NTA Magnetic Agarose Beads; QIAGEN Co.; 36111) were added thereto and allowed to stand at room temperature while being stirred for 40 min. The Ni-NTA beads were precipitated on a magnetic stand similarly as described above, and washed gently with 100 µl of Wash buffer (50 mM NaH₂PO₄, 300 mM NaCl, 20 mM imidazole, 0.05% Tween20 (pH8.0)). After precipitating the beads, 15 µl of Elute buffer (50 mM NaH₂PO₄, 300 mM NaCl, 250 mM imidazole, 0.05% Tween20 (pH8.0)) was added thereto, and the mixture was allowed to stand at room temperature for 1 min to elute the DNA-protein.

### 11. Selection

The DNA-protein eluted in Step 10 was passed through a column filled with biotin-cellulose gel (Sigma), and the elution was collected. Then, the elution was mixed with fluorescein biotin (5-((N-(6-(biotinyl)amino)hexanoyl)amino)pentyl)thioureidyl)fluorescein) (Molecular Probes Co.) in Binding buffer (50 mM phosphate buffer, 100 mM NaCl), and incubated at 25°C for 1 hr. 20 µl of avidin beads (MAGNOTEX-SA; TaKaRa) were added thereto, and was allowed to stand at room temperature for 10min. The beads were then collected with a magnet, and the buffer was exchanged. After washing the beads three times with the Binding buffer, Elution buffer (50mM phosphate buffer, 100 mM NaCl, 50 mM DTT) was added thereto and was allowed to stand for 1 hr at room temperature. After collecting the beads with a magnet at the bottom, the supernatant was recovered.

### 12. DNA amplification of selected molecules by PCR

Two µl aliquot of the DNA-protein recovered in Step 11 was adjusted to a final volume of 50 µl and was subjected to PCR. PCR was carried out using Ω-RT-L (5'-CAACA ACATT ACATT TTACA TTCTA CAACT ACAAG CCACC-3' (SEQ ID NO: 10)) and Ytag-R (5'-TTTCC CCGCC CCCCG TCCT-3' (SEQ ID NO: 11)) as primers, and TaKaRa Ex Taq (Takara) as Taq polymerase, under the condition of: thermal denaturation at 95°C for 2 min, followed by 30 cycles of thermal denaturation at 95°C for 30 sec, annealing at 69°C for 15 sec, and extension at 72°C for 45 sec. After purification with Primer Remover (Edge Science Co.) to remove the primers, the PCR products were precipitated by ethanol.

### 13. PCR for linking T7 promoter region

### T7 promoter was linked to the DNA obtained in Step 12 for conversion to RNA.

Specifically, PCR without any primers was performed using a DNA comprising the promoter sequence and the non-coding sequence of T7 (T7Ω) (SEQ ID NO: 5) to link the T7 promoter, and the product was purified (similarly as described in Step 2).

### 14. Repetition of selection cycle

The DNA obtained in Step 3 described above was returned to Step 3 described above for transcription, and then the cycle of Steps 4 to 13 was repeated three times.

### 15. Sequencing

The PCR products obtained in above-described Step 14 (finally, four cycles of the above-described steps) were sequenced using primers (NewLeft). In addition, the original library and the PCR products obtained in each of the cycles were also sequenced. As a result, it was confirmed that the random sequences converge to a particular sequence. Thus, the PCR products were cloned, and 20 clones were selected and sequenced again. Homologous sequences were extracted according to the result of sequencing.

### 16. Organic synthesis

The homologous DNA sequences obtained in Step 15 were converted to amino acid sequences, and based on the sequences, peptides were synthesized by the solid-phase organic synthesis method. Deprotection, oxidizing, and reducing treatments as described in Step 8 were performed on the peptides. Then, the peptides were cleaved from the solid-phase and collected.

### 17. Binding assay

The dissociation constants of the synthesized peptides were determined by the fluorescence depolarization method to confirm whether the peptides actually have the binding ability to FITC.

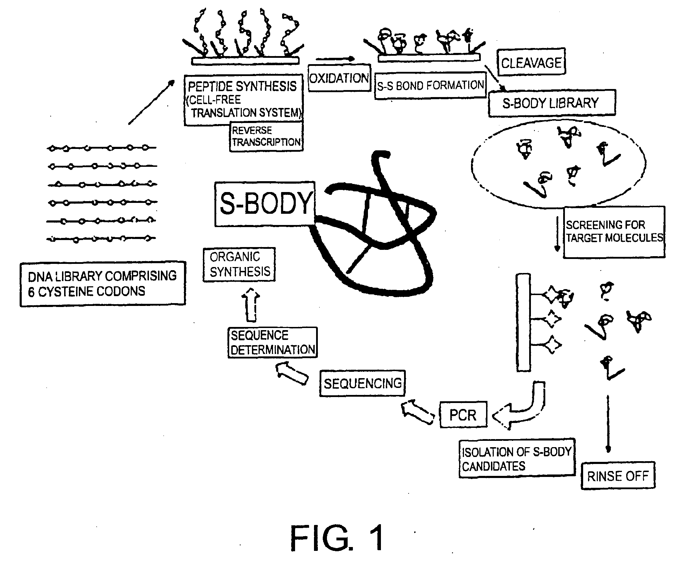

A schematic illustration of the outline of the above-described Example is shown in Fig. 1, as a reference.

### Experimental Example 2: Preparation of binding peptides to fluorescent molecule Cy3

### 1. Synthesis of PM-attached spacer DNAs

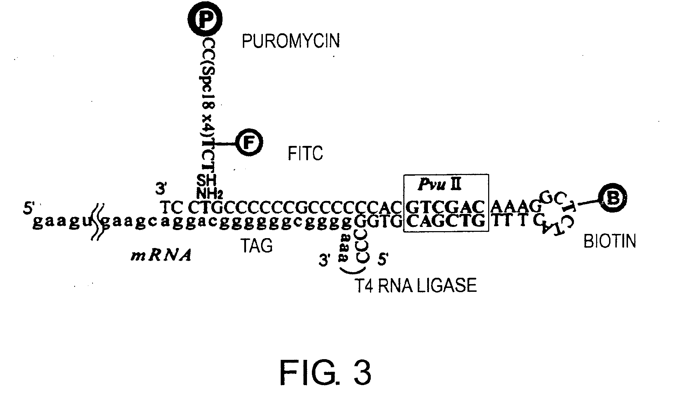

PM-attached spacer DNAs were synthesized according to the method described in Experimental Example 1. Fig. 3 shows the schematic illustration of the method.

### 2. Library construction for in vitro virus synthesis

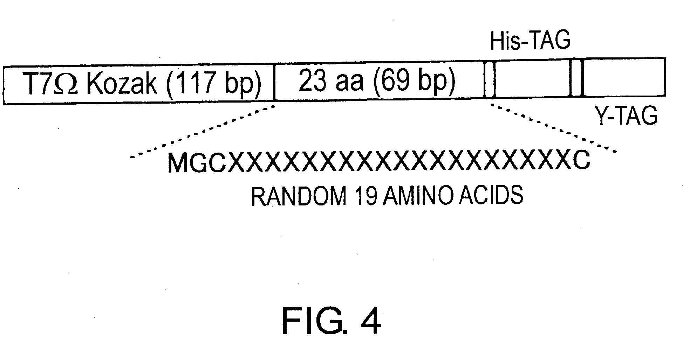

A full-length DNA library comprising random 57-bp sequences was prepared by ligating three single-stranded DNA fragments (SEQ ID NOs: 12 to 14) custom-synthesized at Fasmac Ltd. (Fig. 4). As shown in SEQ ID NOs: 12 to 14, fragments 1 and 2, and fragments 2 and 3, respectively, comprise 20-to 30-mer complementary sequences. Specifically, the full-length DNA library (SEQ ID NO: 15) was obtained by performing extension reactions in the order with fragments 2 and 3, and then with fragment 1. The extension reaction was achieved using Ex Taq (TaKaRa) under the condition of thermal denaturation at 95°C for 2 min, followed by 18 cycles of thermal denaturation at 95°C for 30 sec, annealing at 69°C for 15 sec, and extension at 72°C for 45 sec. The full-length DNA comprises, at the 5'-terminus, the T7 promoter, Cap, omega sequence, and Kozak sequence; and, at the 3'-terminus, the histidine tag and a tag sequence that is complementary, when transcribed, to a portion of the PM-attached spacer DNA (indicated as "Y-tag" in Fig. 4). In the random region from 124-bp to 180-bp of SEQ ID NO: 15, shown as a repetitive sequence of 19 "XYZ", each of the letters indicate nucleotide groups specially mixed to reduce the generation of a stop codon at the time of translation. Specifically, "X" represents a group ofT, C, A, and G of 13%, 20%, 35%, and 32%, respectively; "Y" represents a group of T, C, A, and G of 24%, 22%, 30%, and 24%, respectively; and "Z" represents a group of T, C, A, and G of 37%, 37%, 0%, and 26%, respectively (Protein Sci., 2(8), 1249-54, (1993, Aug.)). In the appended Sequence Listing, this "XYZ" is represented by "nnn". The full-length DNA was excised from denatured acrylamide gel, purified, quantified, and then used for transcription reaction without further treatment.

SEQ ID NO: 12 / Fragment 1 (117 mer)
GATCCCGCGAAATTAATACGACTCACTATAGGGGAAGTATTTTTACAACAATTACCA
ACAACAACAACAAACAACAACAACATTACATTTTACATTCTACAACTACAAGCCAC
CATG

SEQ ID NO: 13 / Fragment 2 (114 mer)
ACATTCTACAACTACAAGCCACCATGGGATGTXYZXYZXYZXYZXYZXYZXYZXYZ
XYZXYZXYZXYZXYZXYZXYZXYZXYZXYZTGTGAGGGGGAGGCAGCCATC
ATC

SEQ ID NO: 14 / Fragment 3 (61 mer)
TTTCCCCGCCGCCCCCCGTCCTGCTTCCGCCGTGATGATGATGATGATGGCTGCCTC
CCCC

SEQ ID NO: 15
GATCCCGCGAAATTAATACGACTCACTATAGGGGAAGTATTTTTACAACAATTACCA
ACAACAACAACAAACAACAACAACATTACATTTTACATTCTACAACTACAAGCCAC
CATGGGATGTXYZXYZXYZXYZXYZXYZXYZXYZXYZXYZXYZXYZXYZ
XYZXYZXYZXYZTGTGAGGGGGGAGGCAGCCATCATCATCATCATCACGGCGGAA
GCAGGACGGGGGGCGGCGGGGAAA

The transcription was carried out using 2 µg of DNA at a scale of 40 µl according to the protocol appended to the kit from Promega Co. (Promega, P 1300). m7G Cap Analog (Promega, P1711) was added for the synthesis. After the mixture was allowed to stand at 37°C for 1 hr, 2 units of DNase (RQ1 DNase) included in the kit were added thereto and then allowed to stand at 37°C for 15 min to digest the template DNAs. After the treatment with phenol and chloroform, the transcription product was purified using DS Primer Remover (Edge Biosystems).

### 3. Conjugation of PM-attached spacer DNA and mRNA

450 pmol of PM-attached spacer DNA and 12 µl of 10x Ligation Buffer (TaKaRa) were added to 450 pmol of the mRNA resulting from the transcription, and DEPC-treated water was added thereto to a total volume of 112 µl. Annealing was performed by changing the temperature from 85°C to 25°C within 10 to 15 min. Then, 24 units of T4 polynucleotide kinase (TaKaRa) and 80 units of T4 RNA ligase (TaKaRa) were added and incubated at 25°C for 40 min. The resulting complex was purified by RNeasy Mini Kit (QIAGEN, 74104).

### 4. Translation (in vitro viral synthesis)

400 pmol of the complex of mRNA and PM-attached spacer DNA was translated at a scale of 2.5 ml using a cell-free translation system (Retic Lysate IVT Kit, Ambion Co; 1200). The translation was carried out at 30°C for 10 min. Then, MgCl₂ and KCl were added at final concentrations of 63 and 750 mM, respectively, and allowed to stand at 37°C for 2 hr to obtain the ternary complex of PM-attached spacer DNA, mRNA, and translated peptide. The scale of translation was, as described above, 400 pmol for the first round of selection, and 50 pmol for the second round. Then, for the subsequent rounds, the translation was performed after a scale-down to 20 pmol.

### 5. Reverse transcription

400-pmol aliquot of the translation product was bound to 400 µl of avidin beads (TaKaRa, 9088, MAGNOTEX-SA) that were previously washed with the appended Binding buffer. Immediately after the washing, the buffer was replaced with 400 µl of reverse transcription buffer (containing 4,000 units of reverse transcriptase M-MLV (TaKaRa, 2640A), 0.5 mM dNTP, and 400 units of RNase inhibitor (Ambion, 2694, SUPERaseIn)) to perform reverse transcription (at 42°C for 10 min) from the nucleotides "CCT" at the 3'-teminus of the PM-attached spacer DNA (SEQ ID NO: 1) to the 5'-terminus of the mRNA.

### 6. Recovery of DNA-protein from beads

To recover 400 pmol of the translation product (the ternary complex of PM-attached spacer DNA, mRNA-DNA conjugate, and translated peptide), and the PM-attached spacer DNA and the mRNA-DNA conjugate that was not used in the translation from the avidin beads, the buffer was replaced with 300 µl of the appended buffer containing 225 units of restriction enzyme *Pvu*II (TaKaRa) and 0.01 % BSA. Then, the mixture was allowed to stand for 1 hr at 37°C.

### 7. His-tag purification

The avidin beads were precipitated to collect the supernatant. To remove the PM-attached spacer DNA-mRNA complex that was not used in the translation, His-tag purification using 100 µl of Ni-NTA beads (QIAGEN, 36111, Ni-NTA Magnetic Agarose Beads) was performed according to the appended protocol. The product purified by the His-tag purification was analyzed by SDS-PAGE (6% gel containing 6 M urea). The confirmation of purity and quantitation was achieved by visualizing the FITC fluorescence of the resulting band using Molecular imager FX (Bio RAD co.), and the product was used for the selection as described below.

### 8. Preparation of Cy3-Sepharose

One vial of Cy3 (Amersham; Q13108; Cy3 monofunctional reactive dye to label 1.0 mg antibody or other protein) was bound to 2 ml of Sepharose gel (Amersham; 17-0569-O1; EAH Sepharose4B) by stirring in 20 mM Hepes (pH7.5) at room temperature for 2 to 6 hr.

### 9. Selection

The purified ternary complex of PM-attached spacer DNA, [mRNA/DNA], and translated peptide was oxidized through shaking on a shaker for 30 min to 2 hr, and leaving standing at 4°C for 1 to 3 days. About 27 x 10¹⁰ and about 3 x 10¹⁰ ternary complexes of PM-attached spacer DNA, [mRNA/DNA], and translated peptide were obtained in the first round and the subsequent rounds, respectively. Almost all of them were used for the selection procedure. The selection was achieved as described below using one dummy column and one Cy3 column in this order for each of the rounds.

First, the purified ternary complex of PM-attached spacer DNA, [mRNA/DNA], and translated peptide was applied to a dummy column (a column (Amersham; 27-3565-01; MicroSpinColumns) filled with Sepharose gel to a column bed volume of about 400 µl). The flow through portion (a volume 1 to 1.5 times greater than the applied volume) was collected. The column had been equilibrated with 10 column volumes or more of Column buffer (10 mM Tris-HCl (pH8.0), 1 M NaCl, 0.1% TritonX100, 1mM EDTA) before use.

Then, the flow through portion collected from the dummy column was bound to Cy3 column (a column filled with Cy3-Sepharose to a column bed volume of about 350 ul) by applying the portion at a flow rate of 0.1 to 0.3 ml/min to the Cy3 column equilibrated with 10 column volumes or more of the Column buffer. After washing with the Column buffer (14 to 63 column volumes (cv)), the ternary complex of PM-attached spacer DNA, [mRNA/DNA], and translated peptide bound to the Cy3 column was eluted (at a flow rate of 0.1 to 0.3 ml/min) with Column buffer containing DTT (Wako, 047-08973, Dithiothreitol) at a final concentration of 50 mM.

The eluted ternary complex of PM-attached spacer DNA, [mRNA/DNA], and translated peptide was desalted, concentrated, and amplified by PCR (using primers 5'-CAACAACATTACATTTTACATTCTACAACTACAAGCCACC-3' (SEQ ID NO: 16) and 5'-TTTCCCCGCCGCCCCCCGTCCTGCTTCCGCCGTGATGAT-3' (SEQ ID NO: 17), under the condition of thermal denaturation at 95°C for 2 min, followed by 30 cycles of thermal denaturation at 95°C for 30 sec, annealing at 69°C for 15 sec, and extension at 72°C for 45 sec). The purified PCR product was ligated with fragment 1 to obtain the full-length DNA again. Then, transcription, ligation with the PM-attached spacer, translation, and purification was performed to use the DNA in the next round of selection.

### 10. Cloning

DNA obtained through consecutive 13 cycles of the selection as described above was ligated with vector pDrive ,(QIAGEN; 231222; PCR Cloning Kit), transformed into QIAGEN EZ Competent Cells, and the cells were cultured on LB plates. 25 colonies were selected at random for DNA sequence analysis. The sequence analysis was performed by Hitachi High-Technologies Corporation Ltd. on order. The nucleotides from 115 bp to 180 bp of all of the analysis results for the 25 clones were converted to amino acids and are shown by one letter codes in SEQ ID NOs: 18 to 42.

Among the 25 clones, 44% comprised N terminal - SCGMLCTHVRHHSRFHMVH (SEQ ID NO: 43), 16% comprised N terminal - SVHFGLQCGNMGHVHDSIH (SEQ ID NO: 44), and 4% comprised N terminal - TLVGSGNPNVGSVIHLHCH (SEQ ID NO: 45).

The sequences selected in the 13th round are shown below. Asterisk (*) in the sequence indicates the site corresponding to a stop codon.

SEQ ID NO: 18: MGCSCGMLCTHVRHHSRFHMVH

SEQ ID NO: 19: MGCSDSARVPLGMAVCVTSSAI

SEQ ID NO: 20: MGCSCGMLCTHVRHHSRFHMVH

SEQ ID NO: 21: MRISRPVMNEGRWLIYLLS

SEQ ID NO: 22: MGRSVHFGLQCGNMGHVHDSIH

SEQ ID NO: 23: MGCSCGMLCTHVRHHSRFHMAN

SEQ ID NO: 24: MGCSCGMLCTHVRHHSHFHMVH

SEQ ID NO: 25: MGCTLVGSGNPNVGSVIHLHCH

SEQ ID NO: 26:    MGCSCGMLCTHVRHHSRFHMVH

SEQ ID NO: 27: MGCSCGMLCTHVRHHSRFHMVH

SEQ ID NO: 28: MGCSCGMLCTHVRHHSRFHMVH

SEQ ID NO: 29:    MGCCNSTGVVVGVLFGPD*MHC

SEQ ID NO: 30: MGCSVHFGLQCGNMGHVHDSIH

SEQ ID NO: 31: MGCSSMSSVHMCFCPAGRDVIS

SEQ ID NO: 32: MGCITFIGECGRFVDGGCFNNN

SEQ ID NO: 33: MGCRARGVGVDYISRRDHKSHH

SEQ ID NO: 34: MGCDLQRVGCAVSATVETCGNS

SEQ ID NO: 35: MGCSCGMLCTHVRHHSRFHMVH

SEQ ID NO:.36:    MGCSVHFGLQCGNMGHVHDSIH

SEQ ID NO: 37: MGCTLVGSGNPNVGSVIHLHCH

SEQ ID NO: 38: MGCSVHFGLQCGNMGHVHDSIH

SEQ ID NO: 39: MGCSCGMLCTHVRHHSRFHMVH

SEQ ID NO: 40: MGCSCGMLRTHVRHHSRFHMVH

SEQ ID NO: 41: MGCISAGDSVCVTDNVDLPSNT

SEQ ID NO: 42:    MGCSCGMLCTHVRHHSRFHMHR

### 11. Determination of association constant to Cy3

As the target molecule was Cy3, which is small and fluorescent, the Kd was determined by the fluorescence depolarization method. However, due to the difficulty in obtaining sufficient amounts of peptides through the above-described cell-free translation system, the measurement was conducted with fluorescence polarization measurement system (BEACON2000; Invitrogen) after obtaining a sufficient amount of peptides (10 µg) through: (1) expression and purification using *E*. *coli;* and (2) peptide synthesis.

### (1) Expression and purification using E. coli

MGC sequence was linked to the N-terminus of the consensus sequence SCGMLCTHVRHHSRFHMVH (SEQ ID NO: 43) obtained through the screening as described above to give MGCSCGMLCTHVRHHSRFHMVH (SEQ ID NO: 18). Furthermore, to the C-terminus of this construct, GGGSGGGS (SEQ ID NO: 46) was ligated as a linker, and [1] glutathione S-transferase (GST) gene or [2] His-tag was linked as the tag sequence (XXX).

Accordingly, the gene region to be inserted into an expression vector was MGCSCGMLCTHVRHHSRFHMVH-GGGSGGGS-XXX. Herein, "XXX" represents either [1] GST (SEQ ID NO: 47) or [2] His-tag (SEQ ID NO: 48).

### <1> GST fusion protein

Peptides were expressed and purified using GST Gene Fusion System (Amersham Bioscience). Complementary DNA strands shown in SEQ ID NOs:49 and 50 having the *Bam*HI site at one end and the *Xho*I site at the other end were prepared to be inserted into expression vector pGEM-1λEcoR1/BAP (Amersham Bioscience).

Specifically, the two DNAs encoding the sequences as described above were synthesized by Fasmac Ltd, and 100 pM each of the DNAs were dissolved in 20 mM Tris-HCl buffer. Hybridization was performed through annealing which was achieved by heating at 95°C for 5 min and then slowly cooling to room temperature in 20 min. The product was treated with restriction enzymes *Bam*HI and *Xho*I*,* and then reacted with pGEM-1λEcoR1/BAP (similarly treated with *Bam*HI and *Xho*I, and then purified) using Quick Ligation Kit (New England Biolab) for 1 hr. *E. coli* cells were transformed with the constructed plasmid to select clones that comprise a plasmid of interest according to the Amersham Bioscience's protocol for expression and detection of GST fusion proteins. The clones were sequenced for confirmation. Then, the *E. coli* cells were cultured according to a general protocol. After the E. *coli* cells were heated in SDS-comprising buffer for 5 min at 95°C, analysis using 12% SDS-PAGE was carried out. After confirmation via CBB staining, the expression of the GST fusion protein was confirmed by Western blotting using goat anti-GST antibody. Then, the protein was purified according to the protocol appended to Bulk GST Purification Module ofAmersham Bioscience. After culturing the E. *coli* cells in 100 ml of culture medium, the culture fluid was centrifuged at 8,000 x g for 10 min. The collected bacterial pellet was suspended in the Starting buffer, and sonicated on ice. Insoluble material was removed by centrifugation at 12,000 x g at 4°C for 10 min. Then, the resulting precipitate was suspended in the Starting buffer. The supernatant and precipitate were subjected to SDS-PAGE to confirm the presence of the protein of interest. Then, glutathione was removed from the buffer using Hitrap Desalting column according to the protocol of GSTrap FF of Amersham Bioscience. In addition to the protein in which the GST tag was removed by cleavage through the procedure as above, a protein that had been purified without the cleavage of the GST tag was prepared.

### <2> His-tag

Peptides were expressed and purified using QIAexpress System (QIAGEN). For cloning into the multi-cloning site of the expression vector pQE-30 Xa (QIAGEN) of this system, *Bam*HI and *Hin*dIII should be placed at the N-terminus and C-terminus, respectively. Thus, complementary DNA stands having the sequences shown in SEQ ID NOs: 51 and 52 were synthesized by Fasmac Ltd. These sequences comprise the respective restriction sites at each of the end of the above-described consensus sequences.

100 pmol each of the synthesized DNAs were dissolved in 20 mM Tris-HCl buffer, heated at 95°C for 5 min, and then hybridized through annealing by slowly cooling to room temperature in 20 min. Then, the resultant was treated with restriction enzymes *Bam*HI and *Hin*dIII*,* and reacted using Quick Ligation Kit (New England Biolab) for 1 hr with pQE-30 Xa (pre-treated with *Bam*HI and *Hin*dIII*,* and then purified). The prepared plasmid was subjected to the following steps according to the protocol of "The QIAexpressionist" of QIAGEN: (i) transformation; (ii) selection of transformants; (iii) determination of the solubility of the objective protein; and (iv) purification under a denaturation condition. Again, in this experiment, in addition to the protein in which the His-tag was removed using Factor Xa, a protein without such removal was prepared.

### (2) Peptide synthesis

Three types of peptides that share the same sequence as indicated below but that differ in S-S cross-linking were ordered from the Peptide Institute, Inc. which has the skills for site specific S-S cross-linking. (C1), (C2), and (C3) are indices of cysteine. MG(C1)S(C2)GML(C3)THVRHHSRFHMVH(SEQ ID NO: 53); MG(C1)S(C2)GML(C3)THVRHHSRFHMVH(SEQ ID NO: 54), wherein (C1) and (C3) are S-S cross-linked; MG(C1)S(C2)GML(C3)THVRHHSRFHMVH(SEQ ID NO: 55), wherein (C1) and (C2) are S-S cross-linked; and MG(C1)S(C2)GML(C3)THVRHHSRFHMVH(SEQ ID NO: 56), wherein (C2) and (C3) are S-S cross-linked.

The yields of these peptides were 1 mg or more.

### (3) Peptide oxidization

The protein purified in (1) was dissolved in 50 mM phosphate buffer (pH7.0), and incubated overnight. Alternatively, the protein was reacted with 50 mM o-iodosobenzoic acid in 50 mM phosphate buffer (pH7) at room temperature for 10 min.

### (4) Fluorescence depolarization assay

Each of the peptide of (2) and the oxidized peptide of (3) was dissolved at a final concentration of 1 µM in 50 mM phosphate buffer. Then, each peptide solution was serially diluted by two-fold. Specifically, the solutions were diluted to concentrations of 500 pM, 250 pM, 125 pM, 62.5 pM, 31.25 pM, 16 pM, 8 pM, 4 pM, 2 pM, 1 pM, 0.5 pM, 0.25 pM, 0.125 pM, and 0.6 pM, and each of the prepared dilution had a volume of 100 µl or more. Cy3 was added thereto at a final concentration of 1 pM. The mixtures were allowed to stand at room temperature for about 10 min, and then successively assayed for depolarization using BEACON2000 (Invitrogen). The obtained data were analyzed using graph analysis software, GraphPad PRISM, to determine Kd.

The assay result showed that each of the samples had a Kd within the range of 1 µM to 100 µM, hence a sufficient binding ability.

### Industrial Applicability

According to the screening methods of the present invention, various proteins forming various tertiary structures can be designed. Further, the proteins can be prepared in a form convenient for evaluating the interaction between the proteins and a target substance, and can be subjected to a screening procedure. Thus, proteins that interact with a proper target substance can be readily screened through the methods of the present invention.

Another embodiment of the present invention has the advantage of allowing the amino acid sequence of an useful protein to be readily optimized via random alteration of the amino acid sequence of the useful protein that interacts with a proper target substance followed by the measurement of the activity of the altered protein. Proteins obtained through such screening methods can be used for various pharmaceutical purposes, as physiologically active substances that modulate the physiological function of a target substance.

## Claims

1. A method of screening for useful proteins by synthesizing a protein comprising a disulfide bond via the introduction of cysteine residues into the amino acid sequence of the protein, and analyzing the function of the protein, wherein the method comprises the steps of:
(a) preparing one or more mRNAs encoding a protein/proteins that comprise at least two cysteine residues, and linking each of the prepared mRNAs with puromycin or a puromycin-like compound to obtain mRNA-puromycin conjugate(s);
(b) contacting a translation system with the mRNA-puromycin conjugate(s) obtained in step (a) to synthesize the protein/proteins, and preparing mRNA-puromycin-protein conjugate(s); and
(c) contacting one or more target substances with the mRNA-puromycin-protein conjugate(s) prepared in step (b), and determining whether the target substances interact with any one of the proteins within the mRNA-puromycin-protein conjugate(s).

2. The method of claim 1, which further comprises, after step (c), the step of:
(d) preparing DNA(s) via reverse transcription of the mRNA(s) of the mRNA-puromycin-protein conjugate(s) comprising the protein(s) that interact with the target substance(s), preparing mRNA(s) having a sequence that is altered by the mutagenesis of the DNA(s), and subjecting the mRNA(s) to step (a) to obtain a protein with enhanced interactive force to the target substance(s).

3. A method of screening for useful proteins by synthesizing a protein comprising a disulfide bond via the introduction of cysteine residues into the amino acid sequence of the protein, and analyzing the function of the protein, wherein the method comprises the steps of:
(a) preparing one or more mRNAs encoding a protein/proteins that comprise at least two cysteine residues, and linking each of the prepared mRNAs with puromycin or a puromycin-like compound to obtain mRNA-puromycin conjugate(s);
(b) contacting a translation system with the mRNA-puromycin conjugate(s) obtained in step (a) to synthesize a protein/proteins, and preparing mRNA-puromycin-protein conjugate(s);
(c) preparing DNA(s) via reverse transcription of the mRNA(s) of the mRNA-puromycin-protein conjugate(s) obtained in step (b), and preparing DNA-puromycin-protein conjugate(s); and
(d) contacting one or more target substances with the DNA-puromycin-protein conjugate(s) prepared in step (c), and determining whether the target substances interact with any one of the proteins of the DNA-puromycin-protein conjugate(s).

4. The method of claim 3, which further comprises, after step (d), the step of:
(e) preparing mRNA(s) having a sequence that is altered by the mutagenesis of the DNA(s) in the DNA-puromycin-protein conjugate(s) comprising the protein(s) that interacts/interact with the target substance(s), and subjecting the mRNA(s) to step (a) to obtain a protein having enhanced interactive force to the target substance(s).

5. The method of any one of claims 1 to 4, wherein, in step (a), the mRNA-puromycin conjugate(s) has/have the structure in which the 3'-terminus of the mRNA(s) is linked to puromycin or the puromycin-like compound via a spacer.

6. The method of any one of claims 1 to 5, wherein, in step (a), the mRNA(s) is/are prepared by transcription of DNA(s) encoding the protein(s) comprising two or more cysteine residues.

7. The method of any one of claims 1 to 6, wherein the translation system used in step (b) is a cell-free translation system.

8. The method of any one of claims 1 to 7, wherein the determination whether the target substance(s) interact with the protein(s) is carried out by evaluating whether the two bind to each other through the affinity column chromatography method or the affinity bead method.

9. The method of any one of claims 1 to 8, which further comprises the step of:
identifying the protein(s) and/or the target substance(s) that have been determined to interact to each other.

10. The method of any one of claims 2 and 4 to 9, wherein the mutagenesis of the DNA is achieved by the error-prone PCR.

11. The method of any one of claims 1 to 10, wherein a protein with enhanced interactive force to the target substance(s) is obtained by repeating each of the steps several times.

12. The method of any one of claims 1 to 11, wherein the mRNA(s) encodes/encode a protein/proteins comprising 8 to 500 amino acid residues.

13. The method of any one of claims 1 to 12, wherein the mRNA(s) encodes/encode a protein/proteins comprising 10 to 200 amino acid residues.

14. The method of any one of claims 1 to 13, wherein the mRNA(s) encodes/encode a protein/proteins comprising 2 to 10 cysteine residues.

15. The method of any one of claims 1 to 14, wherein the mRNA(s) encodes/encode a protein/proteins in which the cysteine residues adjacent to each other are separated in the amino acid sequence with an interval of 2 to 20 residues.

16. The method of any one of claims 1 to 15, wherein the mRNA(s) encodes/encode a protein/proteins in which the cysteine residues positioned to the most N-terminal side and the most C-terminal side are separated by 5 to 50 residues.

17. The method of any one of claims 5 to 16, wherein the spacer comprises, as the major backbone, polynucleotide, polyethylene, polyethylene glycol, polystyrene, peptide nucleic acid, or a combination thereof.

18. The method of any one of claims 5 to 17, wherein the spacer comprises a solid-phase immobilization site and wherein the mRNA-puromycin conjugate(s) is/are linked to a solid phase via the solid-phase immobilization site.

19. The method of claim 18, wherein the solid phase is selected from the group consisting of: styrene bead, glass bead, agarose bead, Sepharose bead, magnetic bead, glass base, silicon base, plastic base, metallic base, glass container, plastic container, and membrane.

20. The method of claim 18 or 19, wherein the solid-phase immobilization site in the spacer has a cleavable site, and wherein the method comprises the step of:
folding the protein(s) on the solid phase and then cleaving the cleavable site.

21. The method of claim 20, wherein the spacer is a DNA spacer and wherein the cleavable site is a restriction enzyme recognition site.

22. A synthetic protein obtainable by the method of any one of claims 1 to 21, which has 8 to 500 amino acid residues, comprises 2 to 10 cysteine residues for forming disulfide bonds, and has an association constant to the target substance that changes due to oxidation and reduction.

23. A synthetic protein which has 8 to 500 amino acid residues, comprises 2 to 10 cysteine residues for forming disulfide bonds, and has an association constant to the target substance that changes due to oxidation and reduction.


SEQUENCE LISTING

<110> GENEFIELD, INC.

<120> METHOD OF SCREENING USEFUL PROTEIN

<130> BVC-A0301Y1P

<150> JP 2003-205139
<151> 2003-07-31

<150> JP 2003-416228
<151> 2003-12-15

<160> 56

<170> PatentIn version 3.1

<210> 1
<211> 55
<212> DNA
<213> Artificial

<220>
<223> an artificially synthesized sequence

<220>

<221> modified_base

<222> (20)..(20)

<223> Biotin is bonded to the 20th cytosine.

<400> 1

cccggtgcag ctgtttcatc cggaaacagc tgcacccccc gccgcccccc gtcct 55

<210> 2

<211> 36

<212> PRT

<213> Artificial

<220>

<223> an artificially synthesized sequence

<220>

<221> MISC_FEATURE

<222> (1)..(4)

<223> "Xaa" = any amino acids.

<220>

<221> MISC_FEATURE

<222> (6)..(8)

<223> "Xaa" = any amino acids.

<220>
<221> MISC_FEATURE
<222> (10)..(12)
<223> "Xaa" = any amino acids.

<220>
<221> MISC_FEATURE
<222> (14)..(17)
<223> "Xaa" = any amino acids.

<220>
<221> MISC_FEATURE
<222> (19)..(22)
<223> "Xaa" = any amino acids.

<220>
<221> MISC_FEATURE
<222> (24)..(31)
<223> "Xaa" = any amino acids.

<220>
<221> MISC_FEATURE
<222> (33)..(36)
<223> "Xaa" = any amino acids.

<400> 2

Xaa Xaa Xaa Xaa Cys Xaa Xaa Xaa Cys Xaa Xaa Xaa Cys Xaa Xaa Xaa

1               5                   10                  15

Xaa Cys Xaa Xaa Xaa Xaa Cys Xaa Xaa Xaa Xaa Xaa Xaa Xaa Xaa Cys

            20                  25                  30

Xaa Xaa Xaa Xaa

        35

<210> 3

<211> 36

<212> PRT

<213> Artificial

<220>

<223> an artificially synthesized sequence

<220>

<221> MISC_FEATURE

<222> (1)..(2)

<223> "Xaa" = any amino acids.

<220>

<221> MISC_FEATURE

<222>  (4)..(12)
<223>  "Xaa" = any amino acids.

<220>
<221>  MISC_FEATURE
<222>  (14)..(15)
<223>  "Xaa" = any amino acids.

<220>
<221>  MISC_FEATURE
<222>  (17)..(21)
<223>  "Xaa" = any amino acids.

<220>
<221>  MISC_FEATURE
<222>  (23)..(27)
<223>  "Xaa" = any amino acids.

<220>
<221>  MISC_FEATURE
<222>  (29)..(31)
<223>  "Xaa" = any amino acids.

<220>
<221>  MISC_FEATURE
<222>  (33)..(36)

<223>  "Xaa" = any amino acids.

<400>  3

Xaa Xaa Cys Xaa Xaa Xaa Xaa Xaa Xaa Xaa Xaa Xaa Cys Xaa Xaa Cys
1               5                   10                  15

Xaa Xaa Xaa Xaa Xaa Cys Xaa Xaa Xaa Xaa Xaa Cys Xaa Xaa Xaa Cys
            20                  25                  30

Xaa Xaa Xaa Xaa
        35

<210>  4
<211>  215
<212>  DNA
<213>  Artificial

<220>
<223>  an artificially synthesized sequence

<220>
<221>  misc_feature
<222>  (71)..(82)
<223>  "n" = a, t, g, or c.

<220>

<221> misc_feature
<222> (86)..(109)
<223> "n" = a, t, g, or c.

<220>
<221> misc_feature
<222> (113)..(124)
<223> "n" = a, t, g, or c.

<220>
<221> misc_feature
<222> (128)..(139)
<223> "n" = a, t, g, or c.

<220>
<221> misc_feature
<222> (143)..(151)
<223> "n" = a, t, g, or c.

<220>
<221> misc_feature
<222> (155)..(163)
<223> "n" = a, t, g, or c.

<220>

<221>  misc_feature
<222>  (167)..(178)
<223>  "n" = a, t, g, or c.

<400>  4
tttccccgcc ccccgtcctg cttccgccgt gatgatgatg atgatggcct ccgcttggag      60

ggccggaggg nnnnnnnnnn nnacannnnn nnnnnnnnnn nnnnnnnnna cannnnnnnn      120

nnnnacannn nnnnnnnnna cannnnnnnn nacannnnnn nnnacannnn nnnnnnnnca      180

tggtggcttg tagttgtaga atgtaaaatg taatg                                 215

<210>  5
<211>  215
<212>  DNA
<213>  Artificial

<220>
<223>  an artificially synthesized sequence

<220>
<221>  misc_feature
<222>  (38)..(43)
<223>  "n" = a, t, g, or c.

<220>

<221> misc_feature

<222> (47)..(73)

<223> "n" = a, t, g, or c.

<220>

<221> misc_feature

<222> (77)..(82)

<223> "n" = a, t, g, or c.

<220>

<221> misc_feature

<222> (86)..(100)

<223> "n" = a, t, g, or c.

<220>

<221> misc_feature

<222> (104)..(118)

<223> "n" = a, t, g, or c.

<220>

<221> misc_feature

<222> (122)..(130)

<223> "n" = a, t, g, or c.

<220>

<221>  misc_feature

<222>  (134)..(145)

<223>  "n" = a, t, g, or c.

<400>  5

catggtggct tgtagttgta gaatgtaaaa tgtaatgnnn nnntgtnnnn nnnnnnnnnn      60

nnnnnnnnnn nnntgtnnnn nntgtnnnnn nnnnnnnnnn tgtnnnnnnn nnnnnnnntg     120

tnnnnnnnnn tgtnnnnnnn nnnnnccctc cggccctcca agcggaggcc atcatcatca     180

tcatcacggc ggaagcagga cggggggcgg ggaaa                                 215

<210>  6

<211>  37

<212>  DNA

<213>  Artificial

<220>

<223>  an artificially synthesized primer sequence

<400>  6

cattacattt tacattctac aactacaagc caccatg                              37

<210> 7

<211> 19

<212> DNA

<213> Artificial

<220>

<223> an artificially synthesized primer sequence

<400> 7

tttccccgcc ccccgtcct 19

<210> 8

<211> 117

<212> DNA

<213> Artificial

<220>

<223> an artificially synthesized primer sequence

<400> 8

gatcccgcga aattaatacg actcactata ggggaagtat ttttacaaca attaccaaca 60

acaacaacaa acaacaacaa cattacattt tacattctac aactacaagc caccatg 117

<210>  9

<211>  19

<212>  DNA

<213>  Artificial

<220>

<223>  an artificially synthesized primer sequence

<400>  9
aggacggggg gcggggaaa                                                    19

<210>  10

<211>  40

<212>  DNA

<213>  Artificial

<220>

<223>   an artificially synthesized primer sequence

<400>  10
caacaacatt acattttaca ttctacaact acaagccacc                             40

<210>  11

<211>  19

<212>  DNA

<213>  Artificial

<220>

<223>  an artificially synthesized primer sequence

<400>  11
tttccccgcc ccccgtcct                                                    19

<210>  12

<211>  117

<212>  DNA

<213>  Artificial

<220>

<223>  an artificially synthesized sequence

<400>  12
gatcccgcga aattaatacg actcactata ggggaagtat ttttacaaca attaccaaca      60

acaacaacaa acaacaacaa cattacattt tacattctac aactacaagc caccatg        117

<210>  13

<211> 114

<212> DNA

<213> Artificial

<220>

<223> an artificially synthesized sequence

<220>

<221> misc_feature

<222> (33)..(89)

<223> "nnn" is repeated 19 times. In the "nnn", 1st n indicates mixtur
e of 13% T, 20% C, 35% A, 32% G, 2nd n indicates mixture of 24% T
, 22% C, 30% A, 24% G, and 3rd n indicates mixture of 37% T, 37%
C, 0% A, 26% G

<400> 13

acattctaca actacaagcc accatgggat gtnnnnnnnn nnnnnnnnnn nnnnnnnnnn 60

nnnnnnnnnn nnnnnnnnnn nnnnnnnnnt gtgagggggg aggcagccat catc 114

<210> 14

<211> 61

<212> DNA

<213> Artificial

<220>

<223>  an artificially synthesized sequence

<400>  14

tttccccgcc gccccccgtc ctgcttccgc cgtgatgatg atgatgatgg ctgcctcccc      60

c                                                                     61

<210>  15

<211>  247

<212>  DNA

<213>  Artificial

<220>

<223>  an artificially synthesized sequence

<220>

<221>  misc_feature

<222>  (124)..(180)

<223>  "nnn" is repeated 19 times.  In the "nnn", 1st n indicates mixtur
       e of 13% T, 20% C, 35% A, 32% G, 2nd n indicates mixture of 24% T
       , 22% C, 30% A, 24% G, and 3rd n indicates mixture of 37% T, 37%
       C, 0% A, 26% G

<400>  15
   gatcccgcga aattaatacg actcactata ggggaagtat ttttacaaca attaccaaca 60
   acaacaacaa acaacaacaa cattacattt tacattctac aactacaagc caccatggga 120
   tgtnnnnnnn nnnnnnnnnn nnnnnnnnnn nnnnnnnnnn nnnnnnnnnn nnnnnnnnnn 180
   tgtgaggggg gaggcagcca tcatcatcat catcacggcg gaagcaggac ggggggcggc 240
   ggggaaa 247

<210> 16

<211> 40

<212> DNA

<213> Artificial

<220>

<223> an artificially synthesized primer sequence

<400> 16

caacaacatt acattttaca ttctacaact acaagccacc 40

<210> 17

<211> 39

<212> DNA

<213>  Artificial

<220>

<223>  an artificially synthesized primer sequence

<400>  17
tttccccgcc gccccccgtc ctgcttccgc cgtgatgat                                39

<210>  18
<211>  22
<212>  PRT
<213>  Artificial

<220>

<223>  A peptide sequence encoded by selected DNA.

<400>  18
Met Gly Cys Ser Cys Gly Met Leu Cys Thr His Val Arg His His Ser
1               5                   10                  15

Arg Phe His Met Val His
            20

<210>  19

<211>  22

<212>  PRT

<213>  Artificial

<220>

<223>  A peptide sequence encoded by selected DNA.

<400>  19

Met Gly Cys Ser Asp Ser Ala Arg Val Pro Leu Gly Met Ala Val Cys

1               5                   10                  15

Val Thr Ser Ser Ala Ile

            20

<210>  20

<211>  22

<212>  PRT

<213>  Artificial

<220>

<223>  A peptide sequence encoded by selected DNA.

<400>  20

Met Gly Cys Ser Cys Gly Met Leu Cys Thr His Val Arg His His Ser

1               5                   10                  15

Arg Phe His Met Val His
            20

<210> 21
<211> 19
<212> PRT
<213> Artificial

<220>
<223> A peptide sequence encoded by selected DNA.

<400> 21
Met Arg Ile Ser Arg Pro Val Met Asn Glu Gly Arg Trp Leu Ile Tyr
1               5                   10                  15

Leu Leu Ser

<210> 22
<211> 22
<212> PRT
<213> Artificial

<220>

<223>  A peptide sequence encoded by selected DNA.

<400>  22

Met Gly Arg Ser Val His Phe Gly Leu Gln Cys Gly Asn Met Gly His

1               5                   10                  15

Val His Asp Ser Ile His

            20

<210>  23

<211>  22

<212>  PRT

<213>  Artificial

<220>

<223>  A peptide sequence encoded by selected DNA.

<400>  23

Met Gly Cys Ser Cys Gly Met Leu Cys Thr His Val Arg His His Ser

1               5                   10                  15

Arg Phe His Met Ala Asn

            20

<210> 24

<211> 22

<212> PRT

<213> Artificial

<220>

<223> A peptide sequence encoded by selected DNA.

<400> 24

Met Gly Cys Ser Cys Gly Met Leu Cys Thr His Val Arg His His Ser

1               5                   10                  15

His Phe His Met Val His

            20

<210> 25

<211> 22

<212> PRT

<213> Artificial

<220>

<223> A peptide sequence encoded by selected DNA.

<400> 25

Met Gly Cys Thr Leu Val Gly Ser Gly Asn Pro Asn Val Gly Ser Val
1               5                   10                  15

Ile His Leu His Cys His
            20

<210> 26
<211> 22
<212> PRT
<213> Artificial

<220>
<223> A peptide sequence encoded by selected DNA.

<400> 26
Met Gly Cys Ser Cys Gly Met Leu Cys Thr His Val Arg His His Ser
1               5                   10                  15

Arg Phe His Met Val His
            20

<210> 27
<211> 22
<212> PRT

<213>  Artificial

<220>
<223>  A peptide sequence encoded by selected DNA.

<400>  27
Met Gly Cys Ser Cys Gly Met Leu Cys Thr His Val Arg His His Ser
1               5                   10                  15

Arg Phe His Met Val His
            20

<210>  28
<211>  22
<212>  PRT
<213>  Artificial

<220>
<223>  A peptide sequence encoded by selected DNA.

<400>  28
Met Gly Cys Ser Cys Gly Met Leu Cys Thr His Val Arg His His Ser
1               5                   10                  15

Arg Phe His Met Val His

            20

<210> 29
<211> 22
<212> PRT
<213> Artificial

<220>
<223> A peptide sequence encoded by selected DNA.

<220>
<221> MISC_FEATURE
<222> (19)..(19)
<223> "Xaa" = The site corresponding to termination codon.

<400> 29
Met Gly Cys Cys Asn Ser Thr Gly Val Val Val Gly Val Leu Phe Gly
1               5                   10                  15

Pro Asp Xaa Met His Cys
            20

<210> 30
<211> 22

<212>  PRT

<213>  Artificial

<220>

<223>  A peptide sequence encoded by selected DNA.

<400>  30

Met Gly Cys Ser Val His Phe Gly Leu Gln Cys Gly Asn Met Gly His

1               5                   10                  15

Val His Asp Ser Ile His

            20

<210>  31

<211>  22

<212>  PRT

<213>  Artificial

<220>

<223>  A peptide sequence encoded by selected DNA.

<400>  31

Met Gly Cys Ser Ser Met Ser Ser Val His Met Cys Phe Cys Pro Ala

1               5                   10                  15

Gly Arg Asp Val Ile Ser
            20

<210> 32
<211> 22
<212> PRT
<213> Artificial

<220>
<223> A peptide sequence encoded by selected DNA.

<400> 32
Met Gly Cys Ile Thr Phe Ile Gly Glu Cys Gly Arg Phe Val Asp Gly
1               5                   10                  15

Gly Cys Phe Asn Asn Asn
            20

<210> 33
<211> 22
<212> PRT
<213> Artificial

<220>

<223>  A peptide sequence encoded by selected DNA.

<400>  33

Met Gly Cys Arg Ala Arg Gly Val Gly Val Asp Tyr Ile Ser Arg Arg

1               5                   10                  15

Asp His Lys Ser His His

            20

<210>  34

<211>  22

<212>  PRT

<213>  Artificial

<220>

<223>  A peptide sequence encoded by selected DNA.

<400>  34

Met Gly Cys Asp Leu Gln Arg Val Gly Cys Ala Val Ser Ala Thr Val

1               5                   10                  15

Glu Thr Cys Gly Asn Ser

            20

<210> 35

<211> 22

<212> PRT

<213> Artificial

<220>

<223> A peptide sequence encoded by selected DNA.

<400> 35

Met Gly Cys Ser Cys Gly Met Leu Cys Thr His Val Arg His His Ser

1               5                   10                  15

Arg Phe His Met Val His

            20

<210> 36

<211> 22

<212> PRT

<213> Artificial

<220>

<223> A peptide sequence encoded by selected DNA.

<400> 36

Met Gly Cys Ser Val His Phe Gly Leu Gln Cys Gly Asn Met Gly His

1               5                   10                  15

Val His Asp Ser Ile His

            20

<210>  37

<211>  22

<212>  PRT

<213>  Artificial

<220>

<223>  A peptide sequence encoded by selected DNA.

<400>  37

Met Gly Cys Thr Leu Val Gly Ser Gly Asn Pro Asn Val Gly Ser Val

1               5                   10                  15

Ile His Leu His Cys His

            20

<210>  38

<211>  22

<212>  PRT

<213>  Artificial

<220>

<223>  A peptide sequence encoded by selected DNA.

<400>  38

Met Gly Cys Ser Val His Phe Gly Leu Gln Cys Gly Asn Met Gly His

1               5                   10                  15

Val His Asp Ser Ile His

            20

<210>  39

<211>  22

<212>  PRT

<213>  Artificial

<220>

<223>  A peptide sequence encoded by selected DNA.

<400>  39

Met Gly Cys Ser Cys Gly Met Leu Cys Thr His Val Arg His His Ser

1               5                   10                  15

Arg Phe His Met Val His

            20

<210>  40

<211>  22

<212>  PRT

<213>  Artificial

<220>

<223>  A peptide sequence encoded by selected DNA.

<400>  40

Met Gly Cys Ser Cys Gly Met Leu Arg Thr His Val Arg His His Ser

1               5                   10                  15

Arg Phe His Met Val His

            20

<210>  41

<211>  22

<212>  PRT

<213>  Artificial

<220>

<223>  A peptide sequence encoded by selected DNA.

<400>  41

Met Gly Cys Ile Ser Ala Gly Asp Ser Val Cys Val Thr Asp Asn Val

1               5                   10                  15

Asp Leu Pro Ser Asn Thr

            20

<210>  42

<211>  22

<212>  PRT

<213>  Artificial

<220>

<223>  A peptide sequence encoded by selected DNA.

<400>  42

Met Gly Cys Ser Cys Gly Met Leu Cys Thr His Val Arg His His Ser

1               5                   10                  15

Arg Phe His Met His Arg

            20

<210>  43

<211>  19

<212>  PRT
<213>  Artificial

<220>
<223>  A peptide sequence encoded by selected DNA.

<400>  43
Ser Cys Gly Met Leu Cys Thr His Val Arg His His Ser Arg Phe His
1               5                   10                  15

Met Val His

<210>  44
<211>  19
<212>  PRT
<213>  Artificial

<220>
<223>  A peptide sequence encoded by selected DNA.

<400>  44
Ser Val His Phe Gly Leu Gln Cys Gly Asn Met Gly His Val His Asp
1               5                   10                  15

Ser Ile His

<210> 45
<211> 19
<212> PRT
<213> Artificial

<220>
<223> A peptide sequence encoded by selected DNA.

<400> 45
Thr Leu Val Gly Ser Gly Asn Pro Asn Val Gly Ser Val Ile His Leu
1               5                   10                  15

His Cys His

<210> 46
<211> 8
<212> PRT
<213> Artificial

<220>

<223>  an artificially synthesized peptide linker sequence.

<400>  46

Gly Gly Gly Ser Gly Gly Gly Ser

1               5

<210>  47

<211>  31

<212>  PRT

<213>  Artificial

<220>

<223>  an artificially synthesized peptide sequence.

<220>

<221>  MISC_FEATURE

<222>  (31)..(31)

<223>  "Xaa" indicates Glutathione S-Transferase.

<400>  47

Met Gly Cys Ser Cys Gly Met Leu Cys Thr His Val Arg His His Ser

1               5                   10                  15

Arg Phe His Met Val His Gly Gly Gly Ser Gly Gly Gly Ser Xaa

            20                  25                  30

<210>  48

<211>  31

<212>  PRT

<213>  Artificial

<220>

<223>  an artificially synthesized peptide sequence.

<220>

<221>  MISC_FEATURE

<222>  (31)..(31)

<223>  "Xaa" indicates His-tag.

<400>  48

Met Gly Cys Ser Cys Gly Met Leu Cys Thr His Val Arg His His Ser
1               5                   10                  15

Arg Phe His Met Val His Gly Gly Gly Ser Gly Gly Gly Ser Xaa
            20                  25                  30

<210>  49

<211>  105

<212>  DNA

<213>  Artificial

<220>

<223>  an artificially synthesized sequence.

<400>  49
gggggatccg gttgctcatg tggcatgcta tgcacacatg ttcggcatca ttcacgattc      60
catatggtgc acggtggtgg atctggtgga gggtctcgaa ttcta                     105

<210>  50
<211>  105
<212>  DNA
<213>  Artificial

<220>

<223>  an artificially synthesized sequence.

<400>  50
tagaattcga gaccctccac cagatccacc accgtgcacc atatggaatc gtgaatgatg      60
ccgaacatgt gtgcatagca tgccacatga gcaaccggat ccccc                     105

<210>  51
<211>  106
<212>  DNA

<213>  Artificial

<220>
<223>  an artificially synthesized sequence.

<400>  51
actggatccg gttgctcatg tggcatgcta tgcacacatg ttcggcatca ttcacgattc      60
catatggtgc acggtggtgg atctggtgga gggtctcaag cttaat                    106

<210>  52
<211>  106
<212>  DNA
<213>  Artificial

<220>
<223>  an artificially synthesized sequence.

<400>  52
attaagcttg agaccctcca ccagatccac caccgtgcac catatggaat cgtgaatgat      60
gccgaacatg tgtgcatagc atgccacatg agcaaccgga tccagt                    106

<210>  53
<211>  22
<212>  PRT

<213>  Artificial

<220>

<223>  an artificially synthesized peptide sequence.

<400>  53

Met Gly Cys Ser Cys Gly Met Leu Cys Thr His Val Arg His His Ser
1               5                   10                  15

Arg Phe His Met Val His
            20

<210>  54

<211>  22

<212>  PRT

<213>  Artificial

<220>

<223>  an artificially synthesized peptide sequence.

<220>

<221>  MISC_FEATURE

<222>  (3)..(3)

<223>  "Cys" indicates the cysteine that binds to 9th amino acid "Cys" by
S-S bond.

<220>

<221>  MISC_FEATURE

<222>  (9)..(9)

<223>  "Cys" indicates the cysteine that binds to 3rd amino acid "Cys" by

S-S bond.

<400>  54

Met Gly Cys Ser Cys Gly Met Leu Cys Thr His Val Arg His His Ser

1               5                   10                  15

Arg Phe His Met Val His

            20

<210>  55

<211>  22

<212>  PRT

<213>  Artificial

<220>

<223>  an artificially synthesized peptide sequence.

<220>

<221>  MISC_FEATURE

<222>  (3)..(3)

<223>  "Cys" indicates the cysteine that binds to 5th amino acid "Cys" by

S-S bond.

<220>

<221>  MISC_FEATURE

<222>  (5)..(5)

<223>  "Cys" indicates the cysteine that binds to 3rd amino acid "Cys" by

S-S bond.

<400>  55

Met Gly Cys Ser Cys Gly Met Leu Cys Thr His Val Arg His His Ser

1               5                   10                  15

Arg Phe His Met Val His

            20

<210>  56

<211>  22

<212>  PRT

<213>  Artificial

<220>

<223>  an artificially synthesized peptide sequence.

<220>

<221>  MISC_FEATURE

<222>  (5)..(5)

<223>  "Cys" indicates the cysteine that binds to 9th amino acid "Cys" by

S-S bond.

<220>

<221>  MISC_FEATURE

<222>  (9)..(9)

<223>  "Cys" indicates the cysteine that binds to 5th amino acid "Cys" by

S-S bond.

<400>  56

Met Gly Cys Ser Cys Gly Met Leu Cys Thr His Val Arg His His Ser

1               5                   10                  15

Arg Phe His Met Val His

            20